# EUROPEAN PATENT SPECIFICATION

(11) **EP 3 661 525 B1**
(45) Date of publication and mention of the grant of the patent: **05.11.2025**
(21) Application number: 18841779.4
(22) Date of filing: 03.08.2018
(51) Int. Cl.: A61K 35/741, A61P 3/08, A61P 3/10

(54) **ROSEBURIA HOMINIS, EUBACTERIUM ELIGENS, AND COMBINATIONS THEREOF AS BIOTHERAPEUTICS**
ROSEBURIA HOMINIS, EUBACTERIUM ELIGENS UND KOMBINATIONEN DAVON ALS BIOTHERAPEUTIKA
ROSEBURIA HOMINIS, EUBACTERIUM ELIGENS ET COMBINAISONS DE CELLES-CI EN TANT QU'AGENTS BIOTHÉRAPEUTIQUES

(30) Priority: 04.08.2017 US 201762541387 P
(43) Date of publication of application: 10.06.2020
(73) Proprietor: GENEVIVE, INC., San Mateo, CA 94402 (US)
(72) Inventor: HAN, Andrew Wonhee, South San Francisco California 94080 (US); GOODYEAR, Andrew Whitman, South San Francisco California 94080 (US); YAMAMOTO, Mitsuko Lynn, South San Francisco California 94080 (US); DESANTIS, Todd Zachary, South San Francisco California 94080 (US); DABBAGH, Karim, South San Francisco California 94080 (US); KAWANA, Shoko, Brisbane, CA 94005 (US); GUJRAL, Tarunmeet, South San Francisco California 94080 (US)
(74) Representative: Fish & Richardson P.C.
(86) International application number: PCT/US2018/045239
(87) International publication number: WO 2019/028402

(56) References cited:
- WO-A1-2013/050792
- WO-A1-2016/019505
- WO-A1-2016/070151
- WO-A1-2016/185469
- WO-A1-2016/203221
- US-A1- 2014 199 281
- US-A1- 2014 363 397
- US-A1- 2016 193 257
- US-A1- 2016 271 188
- US-A1- 2016 366 913
- US-A1- 2017 151 291
- ZOHREH TAMANAI-SHACOORI ET AL: "Roseburia spp.: a marker of health?", FUTURE MICROBIOLOGY, vol. 12, no. 2, 1 February 2017 (2017-02-01), GB, pages 157 - 170, XP055499590, ISSN: 1746-0913, DOI: 10.2217/fmb-2016-0130
- HARIOM YADAV ET AL: "Beneficial Metabolic Effects of a Probiotic via Butyrate-induced GLP-1 Hormone Secretion", JOURNAL OF BIOLOGICAL CHEMISTRY, vol. 288, no. 35, 30 August 2013 (2013-08-30), US, pages 25088 - 25097, XP055303763, ISSN: 0021-9258, DOI: 10.1074/jbc.M113.452516
- AUDREY RIVI�RE ET AL: "Bifidobacteria and Butyrate-Producing Colon Bacteria: Importance and Strategies for Their Stimulation in the Human Gut", FRONTIERS IN MICROBIOLOGY, vol. 7, 28 June 2016 (2016-06-28), XP055703325, DOI: 10.3389/fmicb.2016.00979
- VALCHEVA ROSICA ET AL: "Prebiotics: Definition and protective mechanisms", BAILLIERE'S BEST PRACTICE AND RESEARCH. CLINICAL GASTROENTEROLOGY, BAILLIERE TINDALL, LONDON, US, vol. 30, no. 1, 18 February 2016 (2016-02-18), pages 27 - 37, XP029492207, ISSN: 1521-6918, DOI: 10.1016/J.BPG.2016.02.008

## Description

### FIELD

The present disclosure relates to novel and therapeutically effective compositions and compositions for use in methods for therapeutic treatment comprising live bacteria. The microbial compositions have application, *inter alia,* in regaining healthy glucose homeostasis in a subject. Envisioned are uses of the compositions for use in the treatment, amelioration and/or prevention of diabetes.

### BACKGROUND

The microbiome of the gastrointestinal tract comprises a diverse array of microorganisms, primarily prokaryotes, which play a significant role in the health of the host organism. The complexity of the microbiome, in terms of both its population makeup and composite function, has recently become an intense area of study as research increasingly shows that manipulation of the microbiome can provide health benefits and may be effective in treating a number of diseases and disorders. Currently, a number of probiotics are marketed which contain live bacteria and yeast and are believed to augment the benefits of these microbes which naturally occur in the human body. Increasingly, live biotherapeutic products (LBPs) are being developed for controlled clinical studies and regulatory approval in the treatment of disease. These diseases are multifaceted and present diagnostically in a myriad of ways. WO 2016/070151 discloses methods and compositions relating to microbial treatment and diagnosis of disorders. WO 2016/019505 discloses the use of *Eubacterium* in the prevention and treatment for colorectal cancer related diseases. WO 2013/050792 discloses bacterium for use a probiotic for nutritional and medicinal applications.

There is a great need in the art for the development of a therapeutic, which can not only restore glycemic homeostasis but also treat associated complications thereof in an individual. Research has demonstrated that an improperly functioning glycemic control and regulation can lead to a diverse array of metabolic syndromes, conditions, and/or disorders. The live biotherapeutic products, probiotics, and compositions thereof as taught herein restore a number of human health aspects to a level where homeostasis can be restored and established to prevent or treat a disease, condition, or any slight imbalance in metabolic or inflammatory factors.

### SUMMARY OF THE DISCLOSURE

The present invention is defined by the independent claim. The dependent claims depict other embodiments of the invention. In accordance with the invention, provided herein is a composition for use in a method for treating type 2 diabetes in a subject in need thereof, the composition comprising: i. a therapeutically effective amount of an isolated *Roseburia hominis* (*R. hominis*) bacterial strain; ii. a therapeutically effective amount of an isolated *Eubacterium eligens* (*E. eligens*) bacterial strain; and iii. a pharmaceutically acceptable carrier, said method comprising administering to the subject said composition.

In one embodiment, the subject has presented with a fasting blood glucose level of greater than about 125 mg/dL or greater than about 130 mg/dL.

In one embodiment, the subject has presented with a 2-hour value for a 75-gram oral glucose tolerance test of greater than about 140 mg/dL.

In one embodiment, the method results in a reduction of the subject's fasting blood glucose level of at least about 1%, 2%, 3%, 4%, 5%, 10%, 20%, 30% or 40% of the fasting blood glucose level of the subject prior to the first administration of the composition.

In one embodiment, the reduction of the subject's fasting blood glucose level is measured at 1 month, 2 months, 3 months, 4 months, 5 months, 6 months, 7 months, 8 months, 9 months, 10 months, 11 months, or 12 months after the first administration of the composition.

In one embodiment, the method comprises administering the composition to the subject once, twice or three times per day over a time period of about 1-52 weeks.

In one embodiment, the *R. hominis* further comprises: a 16S rRNA sequence that is at least 97%, 98%, 99%, 99.1%, 99.2%, 99.3%, 99.4%, 99.5%, 99.6%, 99.7%, 99.8%, 99.9% or 100% identical to SEQ ID NO:1, a 16S rRNA gene that is at least 97%, 98%, 99%, 99.1%, 99.2%, 99.3%, 99.4%, 99.5%, 99.6%, 99.7%, 99.8%, 99.9% or 100% identical to SEQ ID NO:2, a 16S rRNA gene that is at least 97%, 98%, 99%, 99.1%, 99.2%, 99.3%, 99.4%, 99.5%, 99.6%, 99.7%, 99.8% 99.9%, or 100% identical to SEQ ID NO:3, and/or a 16S rRNA gene that is at least 97%, 98%, 99%, 99.1%, 99.2%, 99.3%, 99.4%, 99.5%, 99.6%, 99.7%, 99.8%, 99.9% or 100% identical to SEQ ID NO:4.

In some embodiments, the *R. hominis* is strain A2-183 (DSM 16839).

In some embodiments, the *E. eligens* further comprises: a s16s rRNA gene that is at least 97%, 98%, 99%, 99.1%, 99.2%, 99.3%, 99.4%, 99.5%, 99.6%, 99.7%, 99.8%, 99.9% or 100% identical to SEQ ID NO:5, a 16S rRNA gene that is at least 97%, 98%, 99%, 99.1%, 99.2%, 99.3%, 99.4%, 99.5%, 99.6%, 99.7%, 99.8%, 99.9% or 100% identical to SEQ ID NO:6, a 16S rRNA gene that is at least 97%, 98%, 99%, 99.1%, 99.2%, 99.3%, 99.4%, 99.5%, 99.6%, 99.7%, 99.8%, 99.9% or 100% identical to SEQ ID NO:7, a 16S rRNA gene that is at least 97%, 98%, 99%, 99.1%, 99.2%, 99.3%, 99.4%, 99.5%, 99.6%, 99.7%, 99.8%, 99.9% or 100% identical to SEQ ID NO:8, and/or a 16S rRNA gene that is at least 97%, 98%, 99%, 99.1%, 99.2%, 99.3%, 99.4%, 99.5%, 99.6%, 99.7%, 99.8%, 99.9% or 100% identical to SEQ ID NO:9, and/or a 16S rRNA gene that is at least 97%, 98%, 99%, 99.1%, 99.2%, 99.3%, 99.4%, 99.5%, 99.6%, 99.7%, 99.8%, 99.9% or 100% identical to SEQ ID NO:10.

In some embodiments, the *E. eligens* is strain C15-B4 (DSM 3376).

In one embodiment, the *R. hominis* and/or the *E. eligens* in the composition are viable.

In one embodiment, the therapeutically effective amount of *R. hominis* comprises about 1x10⁷ to 1x10¹² colony forming units (CFU).

In one embodiment, the therapeutically effective amount of *E. eligens* comprises about 1x10⁷ to 1x10¹² colony forming units (CFU).

### BRIEF DESCRIPTION OF THE FIGURES

**FIG. 1A** and **FIG. 1B** show effects of treatment with *R. hominis* and *E. eligens* on blood glucose levels **(****FIG. 1A****)** and percent change of blood glucose levels **(****FIG. 1B****)** in an *ob*/*ob* mouse model, as described in Example 1.
**FIG. 2A, FIG. 2B****,** **FIG. 2C,** and **FIG. 2D** show effects of treatment with *R. hominis* and *E. eligens* on glucose homeostasis in an *ob*/*ob* mouse model, as described in Example 2.
**FIG. 3A** and **FIG. 3B** show effects of treatment with *R. hominis* and *E. eligens* on blood insulin levels in a *ob*/*ob* mouse model after two weeks of treatment (Day 15; **FIG. 3A****)** and at the terminus of treatment (Day 22; **FIG. 3B****),** as described in Example 2.
**FIG. 4A** and **FIG. 4B** show effects of treatment with *R. hominis* and *E. eligens* on weight loss **(****FIG. 4A****)** and weight loss percentage **(****FIG. 4B****)** in an *ob*/*ob* mouse model, as described in Example 3.
**FIG. 5** shows effects of administration of *R. hominis* and *E. eligens* on epithelial centric barrier function readouts in a DSS model of inflammatory bowel disease, as described in Example *4. (R. h.* stands for *Roseburia hominis* and *E. e for Eubacterium eligens)*
**FIG. 6** shows effects of administration of *R. hominis* and *E. eligens* on inflammation readouts responsive to impaired barrier function in a DSS model of inflammatory bowel disease, as described in Example 4. *(R. h.* stands for *Roseburia hominis* and *E. e for Eubacterium eligens)*
**FIG. 7** shows effects of administration of *R. hominis* and *E. eligens* on weight loss in a DSS model of inflammatory bowel disease, as described in Example 4. *(R. h.* stands for *Roseburia hominis* and *E. e for Eubacterium eligens)*
**FIG. 8** shows effects of administration of *R. hominis* and *E. eligens* on gross in a DSS model of inflammatory bowel disease, as described in Example 4. *(R. h.* stands for *Roseburia hominis* and *E. e for Eubacterium eligens)*
**FIG. 9** shows effects of administration of *R. hominis* and *E. eligens* on colon weight to length ratio in a DSS model of inflammatory bowel disease, as described in Example 4. *(R. h.* stands for *Roseburia hominis* and *E. e for Eubacterium eligens)*
**FIG. 10** illustrates effects of treatment with *R. hominis* and *E. eligens* on epithelial centric barrier function readouts in an *ob*/*ob* mouse model, as described in Example 5.
**FIG. 11A** and **FIG. 11B** illustrate effects of treatment with *R. hominis* and *E. eligens* on inflammation readouts responsive to impaired barrier function in an *ob*/*ob* mouse model after two weeks of treatment (Day 15; **FIG. 11A****)** and at the terminus of treatment (Day 22; **FIG. 11B****),** as described in Example 5.
**FIG. 12A** and **FIG. 12B** depict effects of treatment with *R*. *hominis* and *E*. *eligens* on small intestine (SI) and colon length, respectively, in an *ob*/*ob* mouse model at the terminus of treatment (Day 22), as described in Example 5.

### DETAILED DESCRIPTION

### Definitions

Unless otherwise defined herein, scientific and technical terms used in this application shall have the meanings that are commonly understood by those of ordinary skill in the art. Generally, nomenclature used in connection with, and techniques of, chemistry, molecular biology, cell and cancer biology, immunology, microbiology, pharmacology, and protein and nucleic acid chemistry, described herein, are those well-known and commonly used in the art. Thus, while the following terms are believed to be well understood by one of ordinary skill in the art, the following definitions are set forth to facilitate explanation of the presently disclosed subject matter.

Throughout this specification, the word "comprise" or variations such as "comprises" or "comprising" will be understood to imply the inclusion of a stated component, or group of components, but not the exclusion of any other components, or group of components.

The term "a" or "an" refers to one or more of that entity, i.e. can refer to a plural referents. As such, the terms "a" or "an," "one or more" and "at least one" are used interchangeably herein. In addition, reference to "an element" by the indefinite article "a" or "an" does not exclude the possibility that more than one of the elements is present, unless the context clearly requires that there is one and only one of the elements.

As used herein the terms "microorganism" or "microbe" should be taken broadly. These terms are used interchangeably and include, but are not limited to, the two prokaryotic domains, Bacteria and Archaea, as well as eukaryotic fungi and protists. In some embodiments, the disclosure refers to the "microbe." This characterization can refer to not only the identified taxonomic bacterial genera of the microbe, but also the identified taxonomic species, as well as the various novel and newly identified bacterial strains.

As used herein, "isolate," "isolated," "isolated microbe," and like terms, are intended to mean that the one or more microorganisms has been separated from at least one of the materials with which it is associated in a particular environment (for example gastrointestinal fluid, gastrointestinal tissue, human digestive fluid, human digestive tissue, etc.). Thus, an "isolated microbe" does not exist in its naturally occurring environment; rather, it is through the various techniques described herein that the microbe has been removed from its natural setting and placed into a non-naturally occurring state of existence. Thus, the isolated strain may exist as, for example, a biologically pure culture, or as spores (or other forms of the strain) in association with a pharmaceutically acceptable carrier suitable for human administration.

In certain aspects of the disclosure, the isolated microbes exist as isolated and biologically pure cultures. As used herein the term "biologically pure" refers to a laboratory culture that is substantially free from other species of organism. Preferably, the bacterial species is in the form of a culture of a single species of organism. It will be appreciated by one of skill in the art, that an isolated and biologically pure culture of a particular microbe, denotes that said culture is substantially free (within scientific reason) of other living organisms and contains only the individual microbe in question. The culture can contain varying concentrations of said microbe. The present disclosure notes that isolated and biologically pure microbes often "necessarily differ from less pure or impure materials." *See, e.g.* In re Bergstrom, 427 F.2d 1394, (CCPA 1970) (discussing purified prostaglandins), see also, In re Bergy, 596 F.2d 952 (CCPA 1979)(discussing purified microbes), see also, *Parke-Davis & Co. v. H.K. Mulford & Co.,* 189 F. 95 (S.D.N.Y. 1911) (Learned Hand discussing purified adrenaline), *aff'd in part, rev'd in part,* 196 F. 496 (2d Cir. 1912). Furthermore, in some aspects, the disclosure provides for certain quantitative measures of the concentration, or purity limitations, that must be found within an isolated and biologically pure microbial culture. The presence of these purity values, in certain embodiments, is a further attribute that distinguishes the presently disclosed microbes from those microbes existing in a natural state. *See, e.g.,* Merck & Co. v. Olin Mathieson Chemical Corp., 253 F.2d 156 (4th Cir. 1958) (discussing purity limitations for vitamin B12 produced by microbes).

In certain aspects of the disclosure, the isolated microbes also encompass the use of mutants or variants of the bacterial species or strains described herein. As used herein, the terms "mutant" and "variant" includes derived bacterial strains having at least 80% identify, at least 85% identify, at least 90% identify, at least 95% identity, at least 98%, or at least 99% identity to the genomic sequence of a referenced strain. Mutants and variants are obtainable by natural processes, mutagenesis campaigns, random culturing, and genetic engineering techniques, among others. The term "mutant" is interchangeable herein with the term "variant."

As used herein, "individual isolates" should be taken to mean a composition, or culture, comprising a predominance of a single genera, species, or strain, of microorganism, following separation from one or more other microorganisms. The phrase should not be taken to indicate the extent to which the microorganism has been isolated or purified. However, "individual isolates" can comprise substantially only one genus, species, or strain, of microorganism.

As used herein, "probiotic" refers to a substantially pure microbe (*i.e.,* a single isolate) or a mixture of desired microbes, and may also include any additional components that can be administered to a subject (*e.g.* a human) for restoring or altering microbiota. Probiotics or microbial inoculant compositions of the disclosure may be administered with an agent to allow the microbes to survive the environment of the gastrointestinal tract, *i.e.,* to resist low pH and to grow in the gastrointestinal environment. In some embodiments, the present compositions (*e.g.*, microbial compositions) are probiotics in some aspects.

As used herein, "prebiotic" refers to an agent that increases the number and/or activity of one or more desired microbes. Non-limiting examples of prebiotics that may be useful in the methods of the present disclosure include fructooligosaccharides (*e.g.*, oligofructose, inulin, inulin-type fructans), galactooligosaccharides, amino acids, alcohols, and mixtures thereof. See Ramirez-Farias et al. (2008. Br. J. Nutr. 4:1-10) and Pool-Zobel and Sauer (2007. J. Nutr. 137:2580-2584 and supplemental).

As used herein, "live biotherapeutic product" or "LBP" refers to a biological product that: 1) contains live organisms, such as bacteria, and 2) is applicable to the prevention, treatment, or cure of a disease or condition of a subject in need thereof. In some embodiments, a LBP is a therapeutic composition which will undergo or has undergone clinical regulatory approval.

A "combination" of two or more bacteria includes the physical co-existence of the bacteria, either in the same material or product or in physically connected products, as well as the temporal co-administration or co-localization of the different bacteria.

The recitations "sequence identity," "percent identity," "percent homology," or for example, comprising a "sequence 50% identical to," as used herein, refer to the extent that sequences are identical on a nucleotide-by-nucleotide basis over a window of comparison. Thus, a "percentage of sequence identity" may be calculated by comparing two optimally aligned sequences over the window of comparison, determining the number of positions at which the identical nucleic acid base (*e.g.,* A, T, C, G, I) occurs in both sequences to yield the number of matched positions, dividing the number of matched positions by the total number of positions in the window of comparison (*i.e.,* the window size), and multiplying the result by 100 to yield the percentage of sequence identity.

As used herein, sequence "identity" can be determined using standard techniques known to those skilled in the art. For example, identity may be determined using the on-line algorithm "BLAST" program, publicly available at blast.ncbi.nlm.nih.gov/Blast.cgi. Alternatively or additionally, to determine the % identity of two nucleic acid sequences, the sequences can be aligned for optimal comparison purposes (*e.g*., gaps can be introduced in one or both of a first and a second sequence for optimal alignment and portions of non-identical sequences can be disregarded as appropriate for comparison purposes). In certain embodiments, the length of a reference sequence aligned for comparison purposes is at least 50%, 60%, 70%, 80%, 90%, 95%, 96%, 97%, 98%, 99% or 100% of the length of the reference sequence. The nucleotides at corresponding nucleotide positions are then compared. The percent identity between the two sequences is a function of the number of identical positions shared by the sequences, taking into account the number of gaps, and the length of each gap, which need to be introduced for optimal alignment of the two sequences.

The phrases "substantially similar" and "substantially identical" in the context of at least two nucleic acids typically means that a polynucleotide comprises a sequence that has at least about 70%, 75%, 80%, 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8% or 99.9% sequence identity, in comparison with a reference polynucleotide.

The "colonization" of a host organism includes the non-transitory residence of a bacterium or other microscopic organism.

The terms "patient," "subject," and "individual" may be used interchangeably and refer to either a human or a non-human animal. These terms include mammals such as humans, non-human primates, livestock animals (*e.g*., bovines, porcines, ovine, caprine, poultry), companion animals (*e.g.,* canines, felines, equine, oryctolagus) and rodents (*e.g.,* mice and rats). In certain embodiments, the terms refer to a human patient. In exemplary embodiments, the terms refer to a human patient that suffers from, e.g., type 2 diabetes, hyperglycemia, hyperinsulinemia, obesity, a gastrointestinal inflammatory condition (*e.g.* IBD), or any combination thereof.

As used herein, "inhibiting and suppressing" and like terms should not be construed to require complete inhibition or suppression, although this may be desired in some embodiments. Thus, an "inhibited immune response" or the "inhibition of inflammatory cytokines" does not require absolute inhibition.

As used herein, the term "hyperglycemia" refers to the condition wherein excess glucose is in the blood stream. For example, fasting blood sugar levels greater than about 125 mg/dL or 130 mg/dL (greater than about 7 mmol/L) can be used to diagnose hyperglycemia in a subject.

As used herein, the term "obesity" refers to the condition characterized by excess body fat. For example, BMI (body mass index, kg/m²) of 30 or above is considered to be obese.

The term "gut" as used herein is meant to refer to the entire gastrointestinal or digestive tract (also referred to as the alimentary canal) and it refers to the system of organs within multicellular animals which takes in food, digests it to extract energy and nutrients, and expels the remaining waste. As used herein the term "gastrointestinal tract" refers to the entire digestive canal, from the oral cavity to the rectum. The term "gastrointestinal tract" includes, but is not limited to, mouth and proceeds to the esophagus, stomach, small intestine, large intestine, rectum and, finally, the anus.

As used herein, the term "therapeutically effective amount" refers to an amount of a therapeutic agent (*e.g.,* a microbe, live biotherapeutic product (LBP), and/or probiotic of the disclosure), which confers a therapeutic effect on the treated subject, at a reasonable benefit/risk ratio applicable to any medical treatment. Such a therapeutic effect may be objective (*i.e.,* measurable by some test or marker) or subjective (*i.e.,* subject gives an indication of, or feels an effect). In some embodiments, "therapeutically effective amount" refers to an amount of a therapeutic agent or composition effective to treat, ameliorate, or prevent (*e.g*., delay onset of) a relevant disease or condition, and/or to exhibit a detectable therapeutic or preventative effect, such as by ameliorating symptoms associated with the disease, preventing or delaying onset of the disease, and/or also lessening severity or frequency of symptoms of the disease.

As used herein, the term "treatment" (also "treat" or "treating") refers to any administration of a therapeutic agent (*e.g.,* a microbe, live biotherapeutic product (LBP), and/or probiotic of the disclosure), according to a therapeutic regimen that achieves a desired effect in that it partially or completely alleviates, ameliorates, relieves, inhibits, delays onset of, reduces severity of and/or reduces incidence of one or more symptoms or features of a particular disease, disorder, and/or condition (*e.g*., chronic or recurring immune response and inflammation of the gastrointestinal (GI) tract, chronic or recurring hyperglycemia); in some embodiments, administration of the therapeutic agent according to the therapeutic regimen is correlated with achievement of the desired effect. Such treatment may be of a subject who does not exhibit signs of the relevant disease, disorder and/or condition and/or of a subject who exhibits only early signs of the disease, disorder, and/or condition. Alternatively or additionally, such treatment may be of a subject who exhibits one or more established signs of the relevant disease, disorder and/or condition. In some embodiments, treatment may be of a subj ect who has been diagnosed as suffering from the relevant disease, disorder, and/or condition. In some embodiments, treatment may be of a subject known to have one or more susceptibility factors that are statistically correlated with increased risk of development of the relevant disease, disorder, and/or condition.

As used herein, the term "medicament" encompasses medicaments for both human and animal usage in human and veterinary medicine. In addition, the term "medicament" as used herein means any substance, which provides a therapeutic and/or beneficial effect. The term "medicament" as used herein is not necessarily limited to substances, which need Marketing Approval, but may include substances which, can be used in cosmetics, nutraceuticals, food (including feeds and beverages for example), probiotic cultures, nutritional supplements and natural remedies. In addition, the term "medicament" as used herein encompasses a product designed for incorporation in animal feed, for example livestock feed and/or pet food.

"Pharmaceutical" implies that a composition, microbe, reagent, method, and the like, are capable of a pharmaceutical effect, and also that the composition is capable of being administered to a subject safely. "Pharmaceutically acceptable" means approved by a regulatory agency of the Federal or a state government or listed in the U.S. Pharmacopoeia or other generally recognized pharmacopoeia for safe use in animals, and more particularly safe use in humans. "Pharmaceutically acceptable vehicle" or "pharmaceutically acceptable excipient" refers to a diluent, adjuvant, excipient or carrier with which a microbe as described herein is administered. The preparation of a pharmaceutical composition or additional active ingredient will be known to those of skill in the art in light of the present disclosure, as exemplified by Remington's Pharmaceutical Sciences, 18th Ed. Mack Printing Company, 1990. Moreover, for animal (*e.g.,* human) administration, it will be understood that preparations should meet sterility, pyrogenicity, general safety and purity standards as required by the FDA Office of Biological Standards.

The therapeutic pharmaceutical compositions taught herein may comprise one or more natural products, however, in certain embodiments, the therapeutic pharmaceutical compositions themselves do not occur in nature. Further, in certain embodiments, the therapeutic pharmaceutical compositions possess markedly different characteristics, as compared to any individual naturally occurring counterpart, or composition component, which may exist in nature. That is, in certain embodiments, the pharmaceutical compositions taught herein-which comprise a therapeutically effective amount of at least one isolated microbe-possess at least one structural and/or functional property that impart markedly different characteristics to the composition as a whole, as compared to any single individual component of the composition as it may exist naturally. The courts have determined that compositions comprising natural products, which possess markedly different characteristics as compared to any individual component as it may exist naturally, are statutory subject matter. Thus, the taught therapeutic pharmaceutical compositions as a whole possess markedly different characteristics. These characteristics are illustrated in the data and examples taught herein.

Details of the disclosure are set forth herein. Although methods and materials similar or equivalent to those described herein can be used in the practice or testing of the present disclosure, illustrative methods and materials are now described. Other features, objects, and advantages of the disclosure will be apparent from the description and from the claims.

### Gastrointestinal Bacteria Beneficial to Metabolic Health

The composition of the intestinal microbiome has been demonstrated to be closely associated with a variety of diseases and disorders including metabolic disease and disorders (Eid et al., 2017, Front Pharmacol, 8:387), cardiovascular disease (Tang et al., 2017, Circ Res, 120:1183-1196), and liver disease (Henao-Mejia et al., 2013, J Autoimmun, 46:66-73) and has been linked to changes in glycemic control (Palau-Rodriguez, 2015, Front Microbiol, 6:1151), in both humans and animals, including animal models.

Glucose homeostasis is the maintenance of glucose tolerance within somewhat narrow physiological limits. An inability to maintain glucose tolerance can contribute to a number of detrimental downstream effects including but not limited to insulin resistance. Insulin resistance is the decreased ability of muscle, fat or liver cells to appropriately use the insulin that has been secreted by pancreatic cells into the bloodstream. As a result, more insulin may be produced to process the same amount of glucose, resulting in hyperinsulinemia. Poor glucose homeostasis in combination with insulin resistance can result in both hyperglycemia and hyperinsulinemia, leading to risk of type 2 diabetes mellitus (T2DM). Short term complications of very high blood sugar levels include ketoacidosis (harmful buildup of ketones in the blood) and hyperosmolar nonketotic syndrome. Long term complications of T2DM include diabetic retinopathy, kidney disease, diabetic neuropathy, and macrovascular problems.

Numerous studies have been carried out to analyze the gut microbiome of persons suffering from a metabolic disorder, including comparisons of the gut microbiome of these persons with that of healthy counterparts. In order to perform such studies, fecal matter from subjects of interest is collected and analyzed to determine the type, and sometimes relative quantity, of bacterial in the feces. The resultant data are indicative of each subject's gut microbiome. Specific and individual bacteria present in a sample are determined by a number of methods involving a combination of molecular and computational elements. Specifically, bacteria may be identified by identification of unique microbial 16S ribosomal RNA (rRNA) sequences (by nucleotide microarray or by sequencing) or through metagenomics analysis which can employ whole genome shotgun sequencing and assembly. The results of each method are dependent upon a number of variables ranging from sample preparation and PCR amplification to software programs used to analyze the laboratory data and parameters selected for each program.

To identify bacterial strains possessing functional importance in the metabolic health of subjects, studies which included the collection and characterization of the gut microbiome from subjects having metabolic disorders were analyzed. Such studies include comparisons of gut microbiota in subjects diagnosed with type 2 diabetes vs. healthy patients (Qin et al.,2012, Nature 490:55-60; Forslund et al., 2015, Nature, 528:262-266), gut microbiota from subjects before and after they had undergone Roux-en-Y gastric bypass surgery (Sweeney and Morton, 2013, JAMA Surg, 148:563-569; Tremaroli et al., 2015, Cell Metab, 22:228-238), and gut microbiota from subjects determined to be healthy or with sub-clinical disease correlated with various metabolic disease markers such as BMI, HbA1c, cholesterol levels, or glucose tolerance (Karlsson et al, 2013, Nature 498:99-103; Zeevi et al, 2015, Cell, 163:1079-1094; Yassour et al, 2016, Genome Med, 8:17). Microbe presence was characterized in terms of both diversity and abundance. 16s rRNA and metagenomic sequence data from selected studies were independently analyzed to identify bacterial species which are more likely to be found in the intestine of healthy subjects compared to those more likely to be found in the intestine of subjects suffering.

Bacterial strains identified as potentially significantly associated with healthy individuals were obtained, cultured and analyzed to test their ability to improve physiologic parameters characteristic of metabolic disorders. From these laboratory studies, it was determined that administration of *R. hominis* and *E. eligens* in a combined composition results in reduced blood glucose levels when administered to *ob*/*ob* mice, an established model for obesity (see Example 1 below). *Ob*/*ob* mice lack leptin and eat excessively. As a result, the mice become obese and exhibit other symptoms such as exhibit hyperphagia, a diabetes-like syndrome of hyperglycemia, glucose intolerance, elevated plasma insulin, subfertility, impaired wound healing, and an increase in hormone production from both pituitary and adrenal glands. Moreover, glucose levels were reduced relative to an untreated control in a glucose tolerance test (GTT). Accordingly, the data demonstrate that a composition comprising a combination of viable *R. hominis* and *E. eligens* is effective in improving glucose homeostasis and reducing glucose in the blood of a treated subject.

Another common way to study effects of treatment on metabolic syndrome related disorders is to use a high fat diet-induced mouse model such as C57BL/6J mice that are fed a diet which is about 60% fat and 20% carbohydrates. The C57BL/6J mice are susceptible to obesity, type 2 diabetes and atherosclerosis when maintained on this high fat diet (Collins et al., 2004, Physiol Behav, 81:243-248).

Additionally, experiments were performed to assess the effects of treatment with *R*. *hominis* and *E. eligens* on intestinal epithelial barrier function. Specifically, mice treated with dextran sodium sulfate (DSS), an agent known to decrease intestinal epithelial barrier integrity, and *ob*/*ob* mice were administered *R. hominis* and *E. eligens* (see Examples 4 and 5, respectively). While no effects of *R. hominis* and *E. eligens* were observed when used to treat the *ob*/*ob* mice, administration of *R. hominis* and *E. eligens* to mice treated with DSS resulted in reduced intestinal epithelial barrier permeability, reduced lipopolysaccharide binding protein (LBP), reduction in loss of body weight due to detrimental effects of DSS and a decreased clinical score - all evidence of beneficial effects of *R. hominis* and *E. eligens* treatment in this mouse model of colitis. Accordingly, provided herein are compositions for use in methods for treating a subject suffering from type 2 diabetes, and also provided is the method comprising administration to the subject a composition comprising *R. hominis* and *E. eligens.*

### Therapeutically Effective Live Bacteria

A bacterial composition useful for treating a subject suffering from a metabolic disorder or inflammatory bowel disease may comprise viable *R. hominis* and *E. eligens,* each of which is further described below.

In some embodiments, the microbes taught herein are identified utilizing 16S rRNA gene sequences. The primary structure of major rRNA subunit 16S comprises a particular combination of conserved, variable, and hypervariable regions that evolve at different rates and enable the resolution of both very ancient lineages such as domains, and more modern lineages such as genera. The secondary structure of the 16S subunit includes approximately 50 helices which result in base pairing of about 67% of the residues. The hypervariable regions can provide species/strain-specific signature sequences useful for bacterial identification. Over the previous few decades, the 16S rRNA gene has become the most sequenced taxonomic marker and is the cornerstone for the current systematic classification of bacteria and archaea (Yarza et al. 2014. Nature Rev. Micro. 12:635-645).

Microbes can be distinguished into a genus based on polyphasic taxonomy, which incorporates all available phenotypic and genotypic data into a consensus classification (Vandamme et al., 1996, Microbiol Rev, 60:407-438). In some embodiments, sequence identity of 94.5% or lower for two 16S rRNA genes is strong evidence for distinct genera, 86.5% or lower is strong evidence for distinct families, 82% or lower is strong evidence for distinct orders, 78.5% is strong evidence for distinct classes, and 75% or lower is strong evidence for distinct phyla. Also, populations that share greater than 90%, 91%, 92%, 93%, 94%, 95%, 96%, 97%, 98%, 99%, or 99.9% identity can be considered to be variants of the same species. Another accepted genotypic method for defining species is to isolate marker genes of the present disclosure, sequence these genes, and align these sequenced genes from multiple isolates or variants.

Another accepted genotypic method for defining species is based on overall genomic relatedness, such that strains which share approximately 70% or more relatedness using DNA-DNA hybridization, with 5°C or less Δ*T*ₘ (the difference in the melting temperature between homologous and heterologous hybrids), under standard conditions, are considered to be members of the same species.

The bacterial strains (*R. hominis* and *E. eligens*) disclosed herein and variants thereof may be characterized in part or in whole by comparing at least one 16S rRNA sequence with a corresponding 16S rRNA sequence of a reference strain genomic sequence. Generally, a bacterial strain genomic sequence will contain multiple copies of 16S rRNA sequences. The 16S rRNA gene sequence has been determined for a large number of strains. GenBank (www.ncbi.nlm.nih.gov/genbank/) has over 20 million deposited sequences, of which over 90,000 are of 16S rRNA genes. Comparison of the bacterial 16S rRNA gene sequence has emerged as a preferred genetic technique and allows for new strains to be identified by comparison of sequences with known bacterial DNA sequences using, e.g., BLAST (blast.ncbi.nlm.nih.gov/Blast.cgi). In short, the comparison of the 16S rRNA sequence allows differentiation between organisms at the genus level across all major phyla of bacteria, in addition to classifying strains at multiple levels, including species and sub-species level.

### Roseburia hominis (R. hominis)

*R. hominis,* a commensal gut anaerobe of the phylogenetic cluster XIVa within the Firmicutes phylum, belongs to a dominant group of bacteria in the human gut and is also a major butyrate producer. Studies have shown that some important genomes of *Roseburia* species are very different, indicating diverse functionality. Bacteria from *Clostridium* Cluster XIVa, including the bacterial species *R. intestinalis, R. hominis* and *E. rectale* (all of which are butyrate producers) can induce very different and distinct effects on gut cells, but can also have synergistic positive effects.

In some embodiments, The *R. hominis* comprises a 16S rRNA gene sequence that is at least 97%, 98%, 99%, 99.1%, 99.2%, 99.3%, 99.4%, 99.5%, 99.6%, 99.7%, 99.8% or 99.9% identical to a *R. hominis* 16S rRNA gene sequence from GenBank accession number CP003040 (also GenBank RefSeq accession number NC_015977). CP003040 contains the genomic sequence of *R. hominis* strain A2-183 (aka A2-183T) available at the Leibniz Institute DSMZ-German Collection of Microorganisms and Cell Cultures under the accession number DSM 16839. *R. hominis* 16S RNA gene sequences are located within GenBank accession number CP003040 at positions 25,465-26,999 (SEQ ID NO:1); 625,894-627,428 (SEQ ID NO:2); 1,035,384-1,036,918 (SEQ ID NO:3) and the complement of 3,095,887-3,097,421 (SEQ ID NO:4). The bacterial species used in Examples 1-5 of the present disclosure is *Roseburia hominis* strain A2-183.

Also contemplated herein is a combination of *E. eligens* with the bacterial species *R*. *hominis* as described in Duncan et al. (2006, Int.J.Syst.Evol.Microbiol, 56:2437-2441) and/or *R*. *hominis* as described in Travis et al. (2015, Genome Announcements 3(6)).

### Eubacterium eligens (E. eligens)

*Eubacterium eligens,* a commensal gut anaerobe of the phylogenetic cluster XIVa within the Firmicutes phylum, belongs to a dominant group of bacteria in the human gut and is also a butyrate producer.

Bacteria from *Clostridium* Cluster XIVa, including the bacterial species *Roseburia intestinalis, Eubacterium siraeum* and *Eubacterium rectale* can induce very different and distinct effects on gut cells but can also have synergistic positive effects.

In some embodiments, The *E. eligens* comprises a *E. eligens* 16S rRNA gene sequence that is at least 97%, 98%, 99%, 99.1%, 99.2%, 99.3%, 99.4%, 99.5%, 99.6%, 99.7%, 99.8% or 99.9% identical to an *E*. *eligens* 16S rRNA gene sequence from GenBank accession number NC_012778. NC_012778 contains a complete genome sequence of *E*. *eligens* (strain C15-B4, available at the Leibniz Institute DSMZ-German Collection of Microorganisms and Cell Cultures under the accession number DSM 3376). The 16S rRNA gene sequences for *E. eligens* are identified, e.g., within GenBank accession number NC_012778 at positions 15,553-17,092 (SEQ ID NO:5); 413,145-414,685 (SEQ ID NO:6); 697,427-698,966 (SEQ ID NO:7); 872,234-873,773 (SEQ ID NO:8); and 2,029,850-2,031,389 (SEQ ID NO:9). GenBank accession number L34420 describes an *E*. *eligens* 16S rRNA sequence, provided herein as SEQ ID NO:10. The bacterial species used in Examples 1-5 of the present disclosure is *E. eligens* strain C15-B4.

Genomic sequences for additional *E. eligens* strains can be found at GenBank accession numbers NZ_CZBU00000000 and NZ_CZBV00000000. In other embodiments, the bacterial species is *E. eligens* as described in Mahowald et al. (2009, Proc Natl Acad Sci USA 106:5859-5864).

The disclosure also encompasses the use of variants of the bacterial species or strains described herein including those obtained by genetic engineering techniques to alter the genetic material of the strains of the disclosure or recombining the genetic material of the strains of the disclosure with other polynucleotides. In order to obtain such variant strains, a person skilled in the art can use standard mutagenesis techniques, such as UV radiation or exposure to mutagenic chemical products. The disclosure further comprises any microbes harboring a synthetically derived genome that shares the aforementioned sequence identity to the sequence of GenBank accession number CP003040 or NC_012778. Thus, the disclosure includes naturally isolated, recombinantly produced, and synthetically derived microbes.

In some embodiments, the variants include mutants or derived bacterial strains having a genomic sequence which is at least 97%, 98%, 99%, 99.5%, 99.9%, 99.99% or 99.999% identical to the polynucleotide sequence of a referenced strain (e.g., *E. eligens* GenBank Acc. No. NC_012778 and/or *R. hominis* GenBank Acc. No. CP003040). Examples of software that can perform sequence comparisons include, but are not limited to, the BLAST package (see Ausubel, et al. 1999 Short Protocols in Molecular Biology, 4th Ed-Chapter 18), BLAST 2 (see FEMS Microbial Lett 1999 174(2): 247-50; FEMS Microbial Lett 1999 177(1): 187-8), FASTA (Altschul, et al. 1990 J Mol Biol 403-410) and AlignX for example.

Accordingly, a microbe of the disclosure comprises a genomic sequence that is at least 90%, 91%, 92%, 93%, 94%, 95%, 96%, 97%, 98%, 99%, 99.5%, 99.9%, 99.99% or 99.999% identical to the sequence depicted in GenBank accession number CP003040 or NC_012778, and also has the functional ability to improve the health of a subject in need thereof as described in more detail below.

In some embodiments, a microbe of the disclosure comprises a genomic sequence that is at least 97%, 98%, 99%, 99.5%, 99.9%, 99.99% or 99.999% identical to the sequence depicted in GenBank accession number CP003040 or NC_012778, and also has the functional ability to reduce blood glucose levels, reduce intestinal epithelial barrier permeability (i.e., increase gastrointestinal epithelial cell barrier function in a subject (or in an in vitro cellular assay), and/or reduce pro-inflammatory cytokines when administered to a subject. In some aspects, a microbe of the disclosure is able to reduce TNF-α and/or IL-23, when administered to the subject. In an additional or alternative embodiment, a composition comprising *R. hominis* and *E. eligens* or variants thereof also has the functional ability to increase anti-inflammatory cytokines, when administered to the subject. For example, the composition according to the present disclosure is able to increase IL-10 in the blood when administered to the subject.

When used to treat a subject suffering from an inflammatory bowel disorder or disease, administration of the composition comprising *R. hominis* and *E. eligens* or variants thereof may also or alternatively prevent weight loss, e.g., as compared to a subject suffering from an IBD who is not administered the composition. In some embodiments, administration of the composition increases the functionality of the gastrointestinal mucous barrier in the subject. In the colon, the outer mucus layer is the habitat for commensal bacterial. The inner mucus layer is impervious to bacteria and is renewed every hour by surface goblet cells. An increase in the mucous layer therefore is a benefit of treatment with a probiotic or live biotherapeutic products (LBP) such as one comprising *R. hominis* and *E. eligens* or variants thereof.

In some embodiments, a microbe of the disclosure comprises a genomic sequence that is at least 97%, 98%, 99%, 99.5%, 99.9% , 99.99% or 99.999% identical to the sequence depicted in GenBank accession number CP003040 or NC_012778, and also has the functional ability to improve at least one side effect, or ameliorate at least one symptom, of inflammatory bowel disease such as Crohn's disease or ulcerative colitis disease, when administered to a patient in need thereof.

In some embodiments, a microbe of the disclosure comprises a genomic sequence that is at least , , , , , 9S%, , 97%, 98%, 99%, 99.5%, 99.9%, 99.99% or 99.999% identical to the sequence depicted in GenBank accession number CP003040 or NC_012778, and also has the functional ability to improve at least one side effect, or ameliorate at least one symptom, or improve and/or regulate glucose homeostasis and/or glycemic control, when administered to a patient in need thereof.

### Therapeutic Uses for R. hominis and E. eligens

An increasing number of studies show that a subject's microbiome can affect or be associated with predisposition to or incidence of a metabolic disorder (for a review, see, e.g., Eid et al., 2017, Front Pharmacol, 8: article 387; Johnson et al., 2017, J Mol Med, 95:1-8; Yang and Kweon, 2016, BMB Rep, 49:536-541). Provided herein are compositions for use in methods for treating type 2 diabetes by administering to a subject suffering from the metabolic disorder a composition that contains both *R. hominis* and *E. eligens* as viable bacteria. The Examples provided below show that administration of these bacteria can reduce blood glucose levels as well as increase glucose tolerance in an animal. This improvement in glucose homeostasis shows that the composition is useful in the treatment of numerous metabolic diseases or disorders as such diseases and disorders are linked to dysfunctional glucose homeostasis. Such metabolic diseases and disorders which can be treated with the compositions comprising *R. hominis* and *E. eligens* as disclosed herein are described in more detail below. It thereby follows that the compositions comprising *R. hominis* and *E. eligens* as disclosed herein are further able to reduce, ameliorate or reverse one or more symptoms of these diseases and disorders.

Accordingly, methods are provided for treating, preventing, or ameliorating at least one symptom of a disease or condition, including: administering a therapeutically or prophylactically effective amount of microbe(s) as described herein to a subject in need thereof, *i.e.,* a subject suffering from, or at risk of developing the disease or condition, or at least one symptom of the disease or condition.

Metabolic syndrome is a term used to refer to a cluster of conditions including increased blood pressure, high blood sugar (hyperglycemia), obesity including excess body fat around the waist, and abnormal cholesterol or triglyceride levels. These conditions tend to occur together and can increase risk of heart disease, stroke and diabetes. Any one of the conditions can indicate a predisposition to a serious disease such as type 2 diabetes or atherosclerosis.

Hyperglycemia, the condition of high blood glucose levels, is a hallmark of altered metabolism. The effects of hyperglycemia are disastrous on multiple levels, as it disrupts a number of crucial processes at the cellular level, as well as having adverse effects in a tissue-specific manner (*e.g.* blindness of eye), on whole organs (*e.g.* kidney disease), and even including systemic host effects (*e.g*. ketoacidosis, coma, death, etc.) manner.

Subjects who suffer from or are predisposed to T2DM and/or obesity can present with fasting glucose levels that are higher than normal, e.g., greater than about 125 mg/dL or 130 mg/dL (see, e.g., Pippitt et al., 2016, Amer Fam Physic, 93:103-109). The fasting glucose level is a measurement of blood glucose in a subject who has not eaten for at least 8 hours. Subjects who may benefit from therapy with *R. hominis* and *E. eligens,* include those who have been determined to have a fasting blood glucose level greater than about 125 mg/dL, 130 mg/dL, 140 mg/dL, 150 mg/dL, 160 mg/dL, 170 mg/dL, 180 mg/dL, 190 mg/dL or 200 mg/dL. Accordingly, envisioned herein are compositions for use in methods to reduce hyperglycemia or glucose blood levels in a subject in need thereof, wherein administration of a composition comprising *R. hominis* and *E*. *eligens* reduces the fasting glucose level in the subject to a level below 125 mg/dL, below 130 mg/dL, below 140 mg/dL, or below 150 mg/dL.

These subjects can also have impaired glucose tolerance, e.g., a two-hour plasma glucose in a 75 g oral glucose tolerance test of about 140-199 mg/dL (7.8 to 11.0 mmol per L). The determination of fasting blood glucose level may have been performed 1-7 days or 1-4 weeks prior to the first administration of a composition comprising *R. hominis* and *E. eligens* to the subject. Subjects who may benefit from therapy with *R. hominis* and *E. eligens* include those who have been determined to have a 2-hour value for a 75 gram oral glucose tolerance test (OGTT) of greater than about 140 mg/dL, 150 mg/dL, 160 mg/dL, 170 mg/dL, 180 mg/dL, 190 mg/dL or 200 mg/dL.

Alternatively, a subject suffering from hyperglycemia can have postprandial or reactive hyperglycemia which occurs after eating. Postprandial or reactive hyperglycemia is a blood glucose level above about 180 mg/dL at a time 1-2 hours after eating.

Symptoms of hyperglycemia can be headaches, increased urination, thirst, nausea, blurred vision, weight loss, fatigue, and coma. Hyperglycemia can be caused by hypoinsulinism, a condition in which the insulin producing β cells of the pancreas fail to manufacture insulin or manufacture and secrete a reduced amount of insulin into the bloodstream. In such cases, levels of sugar in the blood are dramatically increased resulting in hyperglycemia. Hyperglycemia can also be caused by failure of some or all of the available insulin in the blood to bind to the body's cell receptors and/or internalization of insulin in the cells is reduced.

Diabetes mellitus is a metabolic, chemical disorder of the human body primarily involving an inability of the body to properly utilize sugar, *i.e.* glucose, and other chemical compounds involved in the metabolism of the body. It is characterized by an elevation in the concentration of sugar in the blood and by the appearance of sugar in the urine. It is estimated that 1.5 to 2% of the world population suffers from diabetes mellitus of some type. In general terms, diabetes mellitus is classified into three main types, namely, type 1 diabetes, impaired glucose tolerance (IGT) and type 2 diabetes (T2DM). In most cases of type 1 diabetes, the β cells in the pancreas, probably through an autoimmune reaction, cease producing insulin into the bloodstream of the person. Insulin is vitally important because it enables properly utilization and consummation of sugar in the bloodstream as part of the metabolism process.

In impaired glucose tolerance and T2DM, the pancreas continues to produce insulin, but the insulin may fail to bind to the appropriate cell receptors and/or internalization of insulin in the cells is reduced. In such cases, there may be a sufficient level of insulin in the blood, but the ability of the cells to uptake glucose is reduced or non-existent because of reduced internalized insulin.

T2DM is one of the most common causes of hyperglycemia. The large-scale DCCT study (*See* The Diabetes Control and Complications Trial Research Group (1993) N. Engl. J. Med. 329, 977-986) concluded that chronically increased levels of blood glucose are a main reason underlying the development of complications in a number of diabetes conditions. It could clearly be shown by the DCCT study in the USA that chronically increased levels of blood glucose are a main reason for the development of diabetes complications, leading to a decreased life expectancy. Cardiovascular deaths with a risk of coronary heart disease increased by 2- to 4-fold in this population, as one example. Examples for diabetes complications include, but are not limited to, micro- and macrovascular damages that possibly manifest themselves in retinopathies, nephropathies that may or may not lead to blindness, renal failure, and the loss of extremities, and are accompanied by an increased risk of cardiovascular diseases.

The existence of type 1, IGT, or type 2 diabetes in a person is usually determined by an oral glucose tolerance test (OGTT). OGTT is a test in which the fasting individual is given a known amount of glucose (sugar) by mouth, and the blood is tested at intervals thereafter to note the quantity of sugar in the blood. A curve is then constructed from which important information about the individual can be drawn. The glucose tolerance test curve will typically show whether the individual is hyperglycemic (diabetic) or whether the individual has too little sugar in his or her blood and is therefore hypoglycemic. Example 2 below shows that administration of *R. hominis* and *E. eligens* to an *ob*/*ob* mouse increased glucose tolerance as seen by the decrease in glucose AUC (area under the curve).

In view of the ability of *R. hominis* and *E. eligens* administration to reduce glucose levels in the blood of a subject and to increase glucose tolerance (and homeostasis), provided herein are compositions comprising *R. hominis* and *E. eligens* and use of these compositions to treat a subject suffering from type 2 diabetes mellitus. Also provided are compositions for use in methods for decreasing, ameliorating, and/or reversing one or more symptoms of any of the above-listed disorders. Such symptoms include, for example, headaches, blurred vision, increased urination, thirst, nausea, weight loss, fatigue, and coma.

It is understood that the subject can be a human, a non-human primate or other animal, including but not limited to pets such as dogs and cats, livestock such as cows, horses, pigs, goats, rabbits, and other animals such as rodents (mice and rats), or any other animal in need thereof.

Dysbiosis is also known to have a detrimental effect on other physiologies such as inflammation and immunity. Accordingly, it is highly likely that beneficial effects of therapy using probiotics or live biotherapeutic products will extend beyond a single disorder. For example, administration of a composition comprising *R. hominis* and *E. eligens* to treat symptoms related to a metabolic disorder may also have beneficial effects on other disorders such as inflammatory disorders including inflammatory bowels diseases such as Crohn's disease or ulcerative colitis. Although not part of the claimed subject-matter, a method is also disclosed comprising administering a composition comprising *R. hominis* and *E. eligens* to a subject who have been diagnosed with an inflammatory disorder. One example of an inflammatory disorder is inflammatory bowel disease (IBD). IBDs include, e.g., ulcerative colitis (UC) and Crohn's disease (CD). Accordingly, in one aspect, the method comprising administering a composition comprising *R. hominis* and *E. eligens* to a subject diagnosed with an IBD. In other embodiments, the IBD is UC or CD.

One means for testing the efficacy of a composition according to the present disclosure as effective in treating an IBD is an in vitro assay of intestinal epithelial barrier disease. Gastrointestinal epithelial barrier integrity can be measured in in vitro experimental systems using a transepithelial/transendothelial electrical resistance (TEER) assay which measures electrical resistance across a cellular monolayer. This very sensitive and reliable method determines integrity and permeability of the monolayer. Background information on TEER assays is available, e.g., in Dewi, et al. (2004) J. Virol. Methods. 121:171-180, and in Mandic, et al. (2004) Clin. Exp. Metast. 21:699-704. Guidance on transendothelial cell albumin permeability assays is available, e.g., in Dewi, et al. (2008) J. Gen. Virol. 89:642-652. Staurosporine is a reagent that can be used as a control with TEER assays. Also not claimed but disclosed is a method for increasing intestinal epithelial barrier function integrity with a composition comprising *R. hominis* and *E*. *eligens,* wherein the method increases electrical resistance in a TEER assay by at least 10%, 20%, 30%, 40%, 50%, 60%, 70%, 80%, 90% or 100% as compared to the TEER assay run in the absence of *R. hominis* and *E. eligens.*

Therapeutic efficacy of the compositions and compositions for use in methods disclosed herein can encompass a reduction in symptoms associated with the indication being treated. For example, a subject diagnosed with and being treated for type 2 diabetes, may see a reduction is one or more symptoms selected from the group consisting of dry mouth and itchy skin, blurred vision, onset of a yeast infection and pain or numbness in the feet or legs. A reduction in symptoms can be a reduction in the number of times a subject experiences the symptom(s) in a 24-hour period. The reduction in the one or more symptoms can be observed over, e.g., a period of about 1-2 months, 1-5 months, or 6-12 months after the initiation of treatment.

### Compositions comprising R. hominis and E. eligens

The microbe compositions of the present disclosure can be administered to a subject in need thereof to enhance general health and well-being and/or to treat or prevent a disease or disorder such as a metabolic disorder or disorder associated with reduced intestinal epithelial barrier function as described herein. In some embodiments, the microbe composition is a live biotherapeutic product (LBP) while in other embodiments, the microbe composition is a probiotic. In some embodiments, one or both of the microbes (*R. hominis* and *E. eligens*) are isolated and have been cultured outside of a subject to increase the number or concentration of the microbes, thereby enhancing the therapeutic efficacy of a composition comprising the microbe population.

In some embodiments, the microbe composition is in the form of a live bacterial population. The live population may be, e.g., frozen, cryoprotected or lyophilized. In other embodiments, the microbe composition comprises a non-viable bacterial preparation, or the cellular components thereof. In some embodiments, where the microbe composition is in the form of a non-viable bacterial preparation, it is selected from, for example, heat-killed bacteria, irradiated bacteria and lysed bacteria.

In some embodiments, the bacterial species is in biologically pure form, substantially free from other species of organism. In some embodiments, the bacterial species is in the form of a culture of a single species of organism.

Compositions comprising *R. hominis* and *E. eligens* in accordance with the present disclosure can be any of a number of accepted probiotic or live biotherapeutic products (LBP) delivery systems suitable for administration to a subject. Importantly, a composition for delivery of a live population of *R. hominis* and *E. eligens* must be formulated to maintain viability of the microbes. In some embodiments, the composition comprises elements which protect the bacteria from the acidic environment of the stomach. In some embodiments, the composition includes an enteric coating.

In some embodiments, the composition is a food-based product. A food-based product can be, for example, a yogurt, cheese, milk, meat, cream, or chocolate. Such food-based products can be considered edible, which means that it is approved for human or animal consumption.

One aspect of the disclosure relates to a food product comprising the bacterial species defined above. The term "food product" is intended to cover all consumable products that can be solid, jellied or liquid. Suitable food products may include, for example, functional food products, food compositions, pet food, livestock feed, health foods, feedstuffs, and the like. In some embodiments, the food product is a prescribed health food.

As used herein, the term "functional food product" means food that is capable of providing not only a nutritional effect, but is also capable of delivering a further beneficial effect to the consumer. Accordingly, functional foods are ordinary foods that have components or ingredients (such as those described herein) incorporated into them that impart to the food a specific functional-e.g. medical or physiological benefit-other than a purely nutritional effect.

Examples of specific food products that are applicable to the present disclosure include milk-based products, ready to eat desserts, powders for re-constitution with, e.g., milk or water, chocolate milk drinks, malt drinks, ready-to-eat dishes, instant dishes or drinks for humans or food compositions representing a complete or a partial diet intended for humans, pets, or livestock.

In one embodiment, the composition according to the present disclosure is a food product intended for humans, pets or livestock. The composition may be intended for animals selected from the group consisting of non-human primates, dogs, cats, pigs, cattle, horses, goats, sheep, or poultry. In another embodiment, the composition is a food product intended for adult species, in particular human adults.

Another aspect of the disclosure relates to food products, dietary supplements, nutraceuticals, nutritional formulae, drinks and medicaments containing the bacterial species as defined above, and use thereof.

In the present disclosure, "milk-based product" means any liquid or semi-solid milk or whey based product having a varying fat content. The milk-based product can be, e.g., cow's milk, goat's milk, sheep's milk, skimmed milk, whole milk, milk recombined from powdered milk and whey without any processing, or a processed product, such as yoghurt, curdled milk, curd, sour milk, sour whole milk, butter milk and other sour milk products. Another important group includes milk beverages, such as whey beverages, fermented milks, condensed milks, infant or baby milks; flavored milks, ice cream; milk-containing food such as sweets.

The microbe composition can be a tablet, a chewable tablet, a capsule, a stick pack, a powder, or effervescent powder. The composition can comprise coated beads which contain the bacteria. A powder may be suspended or dissolved in a drinkable liquid such as water for administration.

In some embodiments, the microbe composition comprises a microbe which is isolated. The isolated microbe may be included in a composition with one or more additional substance(s). For example, the isolated microbe may be included in a pharmaceutical composition with one or more pharmaceutically acceptable excipient(s).

In some embodiments, the microbe composition may be used to promote or improve human health. In some aspects, the microbe composition may be used to improve gut health. In some aspects, the microbe composition may be used to regulate appetite. In some aspects, the microbe composition may be used to regulate blood glucose levels. In some aspects, the microbe composition may be used to regulate insulin sensitivity. In some embodiments, the disclosed microbe composition is used for regulating appetite in a subject.

The microbes described herein may also be used in prophylactic applications. In prophylactic applications, bacterial species or compositions according to the disclosure are administered to a patient susceptible to, or otherwise at risk of, a particular disease in an amount that is sufficient to at least partially reduce the risk of developing a disease. The precise amounts depend on a number of patient specific factors such as the patient's state of health and weight.

In some embodiments, the disclosure provides for various immediate and controlled release formulations comprising the taught microbes and combinations thereof. Controlled release formulations sometimes involve a controlled release coating disposed over the bacteria. In particular embodiments, the controlled release coatings may be enteric coatings, semi-enteric coatings, delayed release coatings, or pulsed release coatings may be desired. In particular, a coating will be suitable if it provides an appropriate lag in active release (*i.e.* release of the therapeutic microbes and combinations thereof). It can be appreciated that in some embodiments one does not desire the therapeutic microbes and combinations thereof to be released into the acidic environment of the stomach, which could potentially degrade and/or destroy the taught microbes, before it reaches a desired target in the intestines.

In some embodiments, the microbe compositions of this disclosure encompass *R*. *hominis* and *E. eligens* and any variants thereof as described above.

In some embodiments, the microbe composition of the present disclosure further comprises a prebiotic in an amount of from about 1 to about 30% by weight, respect to the total weight composition, preferably from 5 to 20% by weight. Preferred carbohydrates are selected from: fructo-oligosaccharides (or FOS), short-chain fructo-oligosaccharides, inulin, isomalt-oligosaccharides, pectins, xylo-oligosaccharides (or XOS), chitosan-oligosaccharides (or COS), beta-glucans, arable gum modified and resistant starches, polydextrose, D-tagatose, *acacia* fibers, carob, oats, and citrus fibers. Particularly preferred prebiotics are the short-chain fructo-oligosaccharides (for simplicity shown herein below as FOSs-c.c); said FOSs-c.c. are not digestible carbohydrates, generally obtained by the conversion of the beet sugar and including a saccharose molecule to which three glucose molecules are bonded.

In one embodiment, the composition further comprises at least one other kind of other food grade bacterium, wherein the food grade bacterium is preferably selected from the group consisting of lactic acid bacteria, bifidobacteria, propionibacteria or mixtures thereof.

In some embodiments, microbe compositions comprise 10⁶-10¹² CFU (colony forming units), 10⁸-10¹² CFU, 10¹⁰-10¹² CFU, 10⁸-10¹⁰ CFU, or 10⁸-10¹¹ CFU each of *R. hominis* and *E. eligens.* In other embodiments, microbial combinations comprise about 10⁶, about 10⁷, about 10⁸, about 10⁹, about 10¹⁰, about 10¹¹, or about 10¹² CFU each of *R. hominis* and *E. eligens.*

A composition comprising *R. hominis and E. eligens* according to the present disclosure can be formulated for delivery to a desired site of action within an individual to whom it is administered. For example, the composition may be formulated for oral and/or rectal administration. Additionally, the composition may be formulation for administration to the gastrointestinal lumen, or for delayed release in the intestine, terminal ileum, or colon.

When employed as a pharmaceutical, *i.e.,* for treatment or prophylaxis of a disease, disorder, or condition, the compositions described herein are typically administered in the form of a pharmaceutical composition. Such compositions can be prepared in a manner well known in the pharmaceutical art and include at least one active compound, *i.e*., a live strain as described herein. Generally, the compositions are administered in a pharmaceutically effective amount, *i.e.,* a therapeutically or prophylactically effective amount. The amount of the active agent, *i.e.,* a microbe as described herein, administered will typically be determined by a physician, in the light of the relevant circumstances, including the condition to be treated, the chosen route of administration, the activity of the microbe or microbes administered, the age, weight, and response of the individual patient, the severity of the patient's symptoms, and the like.

The compositions can be administered by a variety of routes including oral, rectal, and intranasal. Depending on the intended route of delivery, the compositions are formulated as either injectable or oral compositions, or as salves, as lotions, or as patches.

The compositions for oral administration can take the form of bulk liquid solutions or suspensions, or bulk powders. More commonly, however, the compositions are presented in unit dosage forms to facilitate accurate dosing. Typical unit dosage forms include prefilled, premeasured ampules or syringes of the liquid compositions or pills, tablets, capsules or the like in the case of solid compositions. The above-described components for orally administrable, or injectable administrable compositions are merely representative. Other materials, as well as processing techniques and the like are set forth in Part 8 of Remington's The Science and Practice of Pharmacy, 21st edition, 2005, Publisher: Lippincott Williams & Wilkins.

For oral administration, particular use is made of compressed tablets, pills, tablets, gellules, drops, and capsules.

The compositions may be formulated in unit dosage form, i.e., in the form of discrete portions containing a unit dose, or a multiple or sub-unit of a unit dose.

In another embodiment, the compositions of the disclosure are administered in combination with one or more other active agents. In such cases, the compositions of the disclosure may be administered consecutively, simultaneously or sequentially with the one or more other active agents.

### Dosage and Administration Schedule

The dosages disclosed herein are exemplary of the average case. There can of course be individual instances where higher or lower dosage ranges are merited, and such are within the scope of this disclosure. The term "unit dosage form" refers to a physically discrete unit suitable as a unitary dosage for an individual to whom administered, each unit containing a predetermined quantity of active material calculated to produce the desired therapeutic or prophylactic effect, and may be in association with a suitable pharmaceutical excipient.

In some embodiments, the effective daily dose in a subject is from about 1×10⁶ to about 1×10¹² colony forming units (CFUs), 1×10⁷ to 1×10¹² CFUs, 1×10⁸ to 1×10¹² CFUs, 1×10⁸ to 1×10¹¹ CFUs, 1×10⁸ to 1×10¹⁰ CFUs, 1×10⁸ to 1×10⁹ CFUs, 1×10⁹ to 1×10¹² CFUs, 1×10¹⁰ to 1×10¹² CFUs, or 1×10¹⁰ to 1×10¹¹ CFUs. The subject may be a human or non-human primate. Alternatively, the subject may be another mammal such as a rat, mouse, rabbit, etc.

In some embodiments, the daily dose is administered to the subject daily for about 1 to 2 weeks, 1 to 4 weeks, 1 to 2 months, 1 to 6 months, 1 to 12 months.

Alternatively, the dose which ranges from about 1×10⁶ to about 1×10¹² colony forming units (CFUs), 1×10⁷ to 1×10¹² CFUs, 1×10⁸ to 1×10¹² CFUs, 1×10⁸ to 1×10¹¹ CFUs, 1×10⁸ to 1×10¹⁰ CFUs, 1×10⁸ to 1×10⁹ CFUs, 1×10⁹ to 1×10¹² CFUs, 1×10¹⁰ to 1×10¹² CFUs, or 1×10¹⁰ to 1×10¹¹ CFUs is administered to a subject every other day, once per week, 3 times per week, 5 times per week, once per month, twice per month, 3 times per month, once every 2 months, or 3 times, 4 times or 6 times per year. In these embodiments, the dose can be administered to the subject for a period extending from about 1 to 2 weeks, 1 to 4 weeks, 1 to 2 months, 1 to 6 months, 1 to 12 months.

The dose administered to a subject should be sufficient to treat a disease and/or condition, partially reverse a disease and/or condition, fully reverse a disease and/or condition, or establish a healthy-state microbiome. In some aspects, the dose administered to a subject should be sufficient to prevent the onset of symptoms associated with a metabolic disorder, such as hyperglycemia, type 2 diabetes, or obesity. In other embodiments, the dose is effective to treat or ameliorate the symptoms of an inflammatory disorder. In some embodiments, the inflammatory is an inflammatory bowel disease such as Crohn's disease or ulcerative colitis.

Dosing may be in one or a combination of two or more administrations, e.g., daily, bi-daily, weekly, monthly, or otherwise in accordance with the judgment of the clinician or practitioner, taking into account factors such as age, weight, severity of the disease, and the dose administered in each administration.

In another embodiment, an effective amount can be provided in from 1 to 500 ml or from 1 to 500 grams of the bacterial composition having from 10⁷ to 10¹¹ bacteria per ml or per gram, or a capsule, tablet or suppository having from 1 mg to 1000 mg lyophilized powder having from 10⁷ to 10¹¹ bacteria. Those receiving acute treat-ment can receive higher doses than those who are receiving chronic administration (such as hospital workers or those admitted into long-term care facilities).

The effective dose as described above, can be administered, for example, orally, rectally, intravenously, via a subcutaneous injection, or transdermally. The effective dose can be provided as a solid or liquid, and can be present in one or more dosage form units (e.g., tablets or capsules).

### Combination Therapies

The compositions taught herein comprising a therapeutic microbe may be combined with other treatment therapies and/or pharmaceutical compositions. For example, a patient suffering from a metabolic syndrome or disorder such as hyperglycemia, obesity or type 2 diabetes may already be taking a pharmaceutical prescribed by their doctor to treat the condition. In embodiments, the compositions taught herein, are able to be administered in conjunction with the patient's existing medicines.

In some embodiments, a synergistic effect is achieved upon combining the disclosed therapeutic microbe or combination of microbes with one or more additional therapeutic agents. For example, in some embodiments, administration of both *R. hominis* and *E. eligens* is synergistically more effective than either bacterium alone in reducing fasting blood glucose levels, reducing postprandial blood glucose levels, increasing glucose tolerance, decreasing insulin resistance, increasing intestinal epithelial barrier integrity per an in vitro TEER assay or in vivo assay, or decreasing intestinal inflammation.

The compositions, with or without one or more prebiotics, can be administered with other agents in a combination therapy mode, including anti-microbial agents. Administration can be sequential, over a period of hours or days, or simultaneous.

In one embodiment, the microbial compositions, with or without one or more prebiotics, are included in combination therapy with one or more anti-microbial agents, which include anti-bacterial agents, anti-fungal agents, anti-viral agents, and anti-parasitic agents. In some embodiments, the anti-microbial agent(s) does not kill or inhibit function or growth of *R. hominis* and/or *E. eligens.*

### Patient selection based on microbiome

In some embodiments, subjects are human. In other embodiments, subjects are other animals, including but not limited to non-human primates, pigs, goats, dogs, cows, horses, chickens, mice, rats, and cats.

In addition to identifying subjects that may benefit from therapy comprising administration of *R. hominis* and *E. eligens* by determining, e.g., blood glucose levels, glucose tolerance, and/or BMI, a subject may be identified according to the profile of their gut microbiome. Particular bacterial compositions can be selected for individual subjects or for subjects with particular profiles. For example, 16S rRNA sequencing can be performed for a given subject to identify the bacteria present in his or her microbiota. The 16S rRNA sequencing can be used with other methods, e.g., microprofiling, for determining a subject's microbiome profile. The sequencing can either profile the subject's entire microbiome using 16S sequencing (to the family, genera, or species level), a portion of the subject's microbiome using 16S sequencing, or it can be used to detect the presence or absence of specific candidate bacteria that are biomarkers for health or a particular disease state, such as markers of multi-drug resistant organisms or specific genera of concern. Based on the biomarker data, a particular composition can be selected for administration to a subject to supplement or complement a subject's microbiota in order to restore health or treat or prevent disease. In another embodiment, subjects can be screened to determine the composition of their micro biota to determine the likelihood of successful treatment.

### Kits

In certain aspects, the disclosure relates to kits for the treatment of a metabolic or inflammatory disorder or disease. The kits comprise a microbial composition according to the present disclosure. In some embodiments, the kit further comprises a prebiotic, a second therapeutic agent as described herein, or a combination thereof.

The kits provided may comprise one or more containers. The containers may comprise singly isolated microbial compositions comprising one or more microbes and/or singly isolated prebiotic compositions comprising one or more carbohydrates. The microbial compositions, with or without one or more prebiotics, in the different containers may be administered at the same time or at different times, and may be administered in a specific order.

The microbial composition, with or without one or more prebiotics, may comprise live microbes, microbes that are lyophilized, freeze-dried, and/or substantially dehydrated, or the composition may comprise bacterial spores.

The following examples are intended to illustrate, but not limit, the disclosure.

### EXAMPLES

The following experiments utilize a robust mixture of *in vivo* experiments utilizing models obesity/diabetes to demonstrate the therapeutic ability of the taught microbes, alone and in combination, and methods thereof. A commonly used animal model to mimic human obesity, hyperglycemia, type 2 diabetes, and associated conditions and diseases is the use of the *ob*/*ob* mouse model (s*ee, e.g.,* Lindström, P. (2007) Scientific World Journal. 7: 666-685 for review). In addition, a high fat diet-induced mouse model can be used such as C57BL/6J mice that are fed a diet which is about 60% fat and 20% carbohydrates (Collins et al., 2004, Physiol Behav, 81:243-248).

To study effects of live strains on IBD and other colonic inflammation disorders, there are numerous and variable animal models that resemble several features of IBD. Reviews of these models can be found, e.g., in Westbrook et al., (2016) Arch Toxicol 90:2109-2130 and Valatas et al. (2013) Am J Physiol Gastrointest Liver Physiol 305:G763-G785. Animal models of colitis include those arising spontaneously in susceptible strains of certain species (congenic) or through crossbreeding, genetically engineered models generated through knock-out and transgenic technologies, transfer models in which lymphocyts are transferred to lymphopenic mice, and chemically induced models. to those requiring administration of specific concentrations of colitis-inducing chemicals, such as dextran sulphate sodium (DSS). Chemical-induced models of gut inflammation are the most commonly used and best described models of IBD.

The following experiments utilize a robust mixture of *in vivo* experiments utilizing animal models of obesity/diabetes and IBD to demonstrate the therapeutic ability of the taught microbes, alone and in combination, and methods thereof.

### Example 1

### Roseburia hominis (R. hominis) and Eubacterium eligens (E. eligens) improve hyperglycemia in ob/ob mice

The following experiments involve use of an in vivo model, the *ob*/*ob* mouse, to demonstrate the therapeutic ability of a microbial composition comprising *R. hominis* and *E*. *eligens* as disclosed herein to treat metabolic disorders. The *ob*/*ob* mouse model develops severe insulin resistance and is used to mimic human metabolic disorder conditions such as obesity, type 2 diabetes, and diseases and conditions associated with diabetes (e.g. hyperglycemia).

Experiement was performed to show the effects of treatment on blood glucose levels. The *ob*/*ob* mice were treated as follows:

| **Procedure** | **Day** |
|---|---|
| Acclimation | -7 to -1 |
| Fecal Sample Collection | -2 |
| Begin Treatment | 0 |
| FITC-dextran | 15 |
| Fecal Sample Collection | 18 |
| OGTT (oral glucose tolerance test) | 20 |
| End Treatment | 22 |

Beginning on Day 0, mice were treated orally (p.o.) with a total volume of: 1) 0.1 ml PBS containing 2×10⁷ colony forming units (CFU) of *Roseburia hominis* strain A2-183 (DSM/DSMZ No. 16839) and 1×10⁸ CFU of *Eubacterium eligens* strain C15-B4 (DSM/DSMZ No. 3376); 2) 0.1 ml PBS containing *Akkermansia muciniphila* (ATCC deposit No. BAA-835) also referred to as *A. muciniphila* or Akkermansia throughout this disclosure); 3) 0.1 ml PBS containing a vehicle as the negative control; or 4) metformin as a positive treatment control. Treatments continued bi-daily through day 22, oral glucose tolerance tests were performed on day 20, and following 22 days of treatment, mice were euthanized.

*A. muciniphila,* used as a control strain, is known to improve glucose metabolism. See, e.g., Liu et al., Oncotarget, Jan. 17 2017, 8(3):37987-3810; and Greer et al., Nat Commun., Nov. 14, 2016, 7:13329.

Metformin (N,N-dimethylimidodicarbonimidic diamide) is the most prescribed pharmacotherapy for the treatment of individuals with type 2 diabetes. See, e.g., Sharma et al. (2016) BJM Open 6:e010210. Metformin lowers both basal and postprandial plasma glucose and functions by decreasing hepatic glucose production and intestinal absorption of glucose and increasing peripheral glucose uptake and utilization. Metformin improves glucose tolerance in patients with T2D. With metformin therapy, insulin secretion remains unchanged while fasting insulin levels and day-long plasma insulin response may decrease. Metformin has been shown to be associated with higher relative abundance of *A. mucinophilia* in the gut. See, e.g., de la Cuesta-Zuluaga, Jan. 2017, 40(1):54-62.

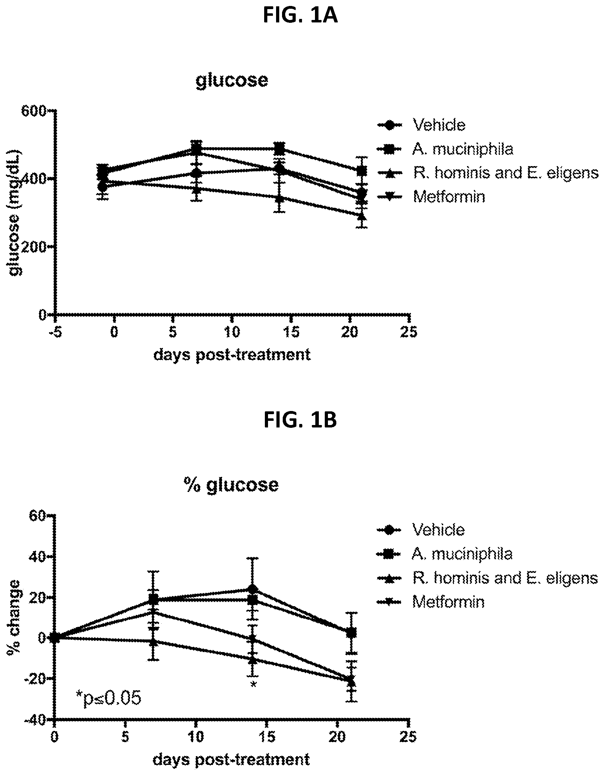

Fed blood glucose levels were measured throughout treatment. Results are shown in **FIG. 1A** and are presented as mean ± SEM. Percent change in blood glucose levels was also determined for each mouse. These results are shown in **FIG. 1B** and are also presented as mean ±

### SEM.

Treatment with *R. hominis* and *E. eligens* led to significantly lower glucose levels and percent decrease in glucose levels than treatment with vehicle or metformin. Glucose levels also decreased sooner after treatment with *R. hominis* and *E. eligens* than after treatment with metformin. *I.e.,* the decrease in glucose levels (and the percent decrease in glucose levels) are drastically greater in the first post-treatment data point, collected at Day 7.

Data from Day 7, for example, show that the average fed glucose levels are 416, 489, 371, and 477 mg/dL in the vehicle, *A. mucinophilia, R. hominis* and *E*. *eligens,* and metformin groups, respectively. At Day 14 post treatment, mice treated with *R. hominis* and *E. eligens* showed a significant change in glucose compared to vehicle treated mice as determined by 2-way ANOVA (n=8-10).

### Example 2

### R. hominis and E. eligens improve hyperglycemia and glucose homeostasis in ob/ob mice

Experiments were done to show the effects of treatment on glucose homeostasis in *ob*/*ob* mice by performing oral glucose tolerance tests (OGTTs) and measuring insulin levels. The *ob*/*ob* mice were those treated as described in Example 1 above.

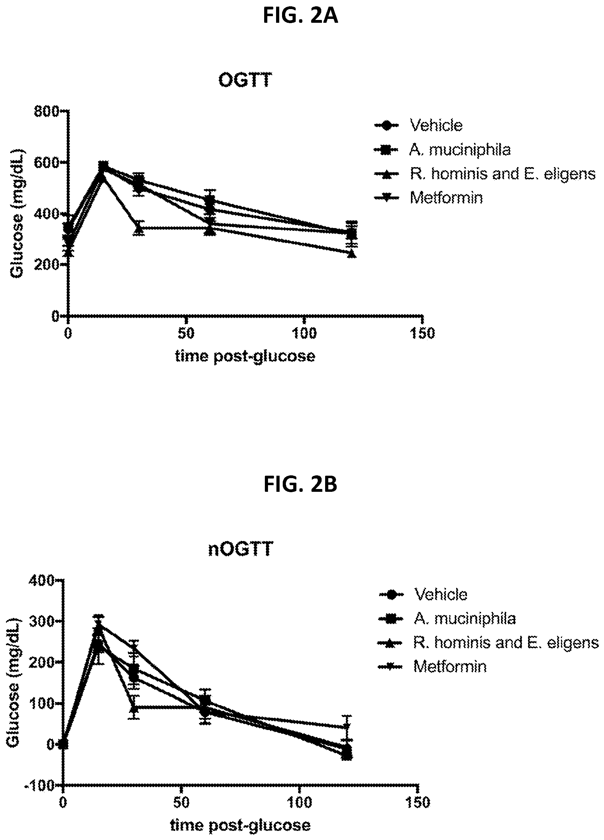

Treatment with *R. hominis* and *E. eligens* led to significantly improved glucose homeostasis in *ob*/*ob* mice. Results are shown in **FIGS. 2A-2D****.** OGTTs were performed after a 5 hour fast. Mice were given 1g/kg glucose by oral gavage and circulating glucose levels were measured over 2 hours from a tail clipping. Statistics were determined by 2-way ANOVA and Fisher's LSD test. Area under the curve (AUC) was determined using GraphPad Prism.

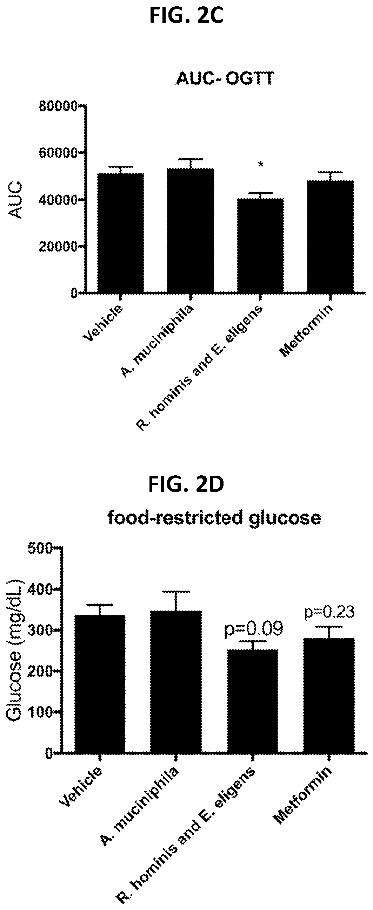

Treatment with *R. hominis* and *E. eligens* led to a significant improvement in glucose tolerance as measured by an OGTT. Results are presented in **FIG. 2A** (OGTT) and **FIG. 2B** (normalized OGTT) and show that treatment with *R. hominis* and *E. eligens* leads to a more rapid reduction in blood glucose levels after glucose administration. Moreover, after 30 minutes, circulating glucose levels were significantly lower in *R. hominis* and *E. eligens-*treated mice compared to the vehicle group. The AUC was accordingly significantly decreased compared to vehicle-treated mice (see **FIG. 2C****).** The AUC was not significantly improved with any other treatment. **FIG. 2D** shows blood glucose levels in the animals after a 5-hour fast and just prior to dosing each animal with glucose. Mice treated with *R. hominis* and *E. eligens* showed a significantly lower blood glucose level compared with vehicle-treated mice.

The study further examined the effects of treatment with *R. hominis* and *E. eligens* on insulin levels in *ob*/*ob* mice. The *ob*/*ob* mice were those treated as described in Example 1 above. Statistics were determined by Fisher's LSD test.

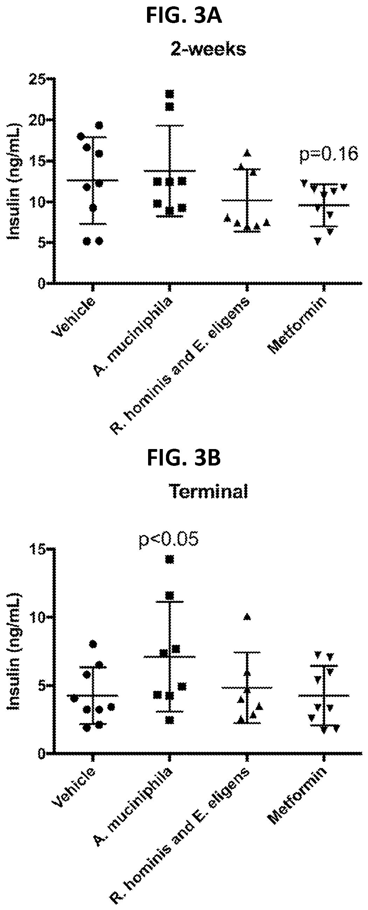

Insulin levels were measured after 2 weeks of treatment (Day 15) and at the termination of the study (3 weeks; Day 22). Insulin in the serum was measured by ELISA. The results are presented in **FIG. 3A** (Day 15) and **FIG. 3B** (Day 22). While no significant effects of treatment on insulin were observed at 2 weeks, at the end of the study, insulin levels were significantly higher in *A. muciniphila-*treated mice compared to vehicle-treated mice. Insulin levels in the metformin and the *R. hominis* and *E. eligens* groups were not significantly different from that of the vehicle, suggesting that the increased glucose tolerance observed in animals treated with *R. hominis* and *E. eligens* was not due to increased insulin levels.

### Example 3

### R. hominis and E. eligens effect on weight loss in ob/ob mice

Example 3 shows effects of treatment on weight change in the *ob*/*ob* mouse model. The *ob*/*ob* mice were those treated as set forth in Example 1 above.

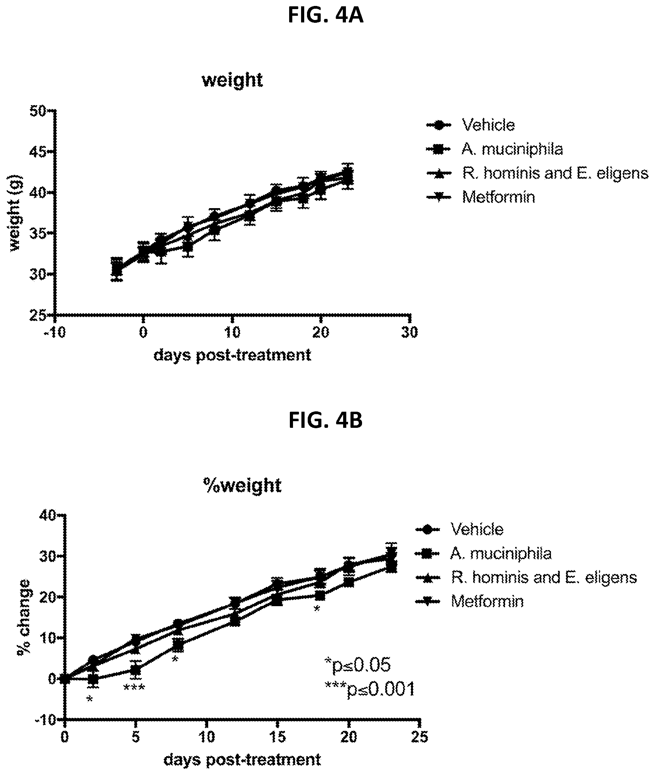

The body weight of each mouse was measured throughout the treatment and results are provided in **FIG. 4A** as mean ± SEM. Percent change from starting weight on day zero was also determined for each mouse. These results are shown in **FIG. 4B** and are also presented as mean ± SEM.

Treatment with *A. mucinophilia* generally led to decreases in both overall weight gain and percent increase in weight. *R. hominis* and *E. eligens* administration did not lead to significant decreases in weight or percent weight increase in this model. Weight loss in mice treated with *R. hominis* and *E. eligens* was similar to weight loss observed with metformin and vehicle only. Statistical differences between groups were determined by a repeated measures 2-way ANOVA (n = 8).

### Example 4

### R. hominis and E. eligens improve epithelial centric barrier function readouts in a DSS model of inflammatory bowel disease

The following experiment demonstrates the therapeutic ability of a microbial composition comprising *R. hominis* and *E. eligens* as disclosed herein to treat epithelial barrier function disorders or related metabolic disorder in an *in vivo* model (C57BL/6 mice treated with dextran sodium sulfate (DSS).

To model inflammatory bowel disease associated with decreased epithelial barrier function *in vivo,* C57BL/6 mice were treated with 2.5% dextran sodium sulfate (DSS) in their drinking water for six days (See, e.g., Chassaign et al., 2014, Curr Protoc Imunol, 104:Unit-15.25; Kiesler et al., 2015, Cell Mol Gastroenterol Hepatol). On Day zero: 1) mice were treated orally (p.o.) with a total volume of 0.2 ml PBS containing 4.7×10⁷ colony forming units (CFU) of *Roseburia hominis* strain A2-183 (DSM/DSMZ No. 16839) and 2.2 × 10⁸ CFU of *Eubacterium eligens* strain C15-B4 (DSM/DSMZ No. 3376); 2) Gly2-GLP treated mice received 50 nmoles/kg Gly2-GLP2 reconstituted in 0.1 ml PBS + 10% glycerol intraperitoneally (i.p.); and 3) vehicle treated mice received 0.1 ml PBS + 10% glycerol i.p. Six hours later, DSS treatment was initiated and mice received 2.5% DSS in their drinking water for the next 6 days. *R. hominis* and *E*. *eligens,* Gly2-GLP2 and vehicle treatments continued bi-daily for the duration of the DSS exposure. Untreated mice were maintained on normal drinking water and did not receive any treatments throughout the entire course of the experiment. On day six, mice were fasted for four hours and then orally gavaged with 600 mg/kg 4KDa dextran labeled with fluorescein isothiocyanate (4KDa-FITC). One hour after the 4KDa-FITC, gavage mice were euthanized, blood was collected, and FITC signal was measured in serum.

### A. Effects of R. hominis and E. eligens on intestinal epithelial barrier function

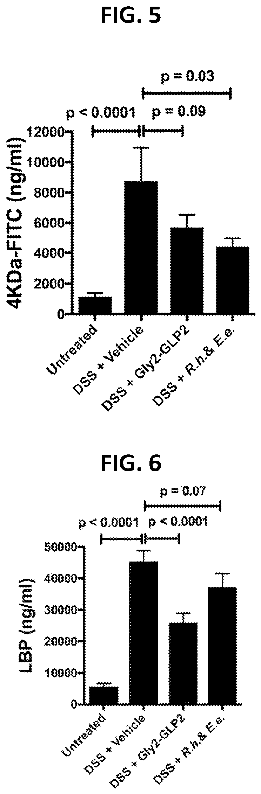

Results are shown in **FIG. 5** and are presented as mean ± SEM. A significant increase in 4KDa-FITC dextran translocation across the epithelial barrier was observed in vehicle-treated DSS mice (p < 0.0001) as compared to untreated mice. Treatment with *R. hominis* and *E*. *eligens* resulted in a significant reduction in 4KDa-FITC dextran translocation as compared to DSS mice treated with Vehicle only (p = 0.03). The magnitude of 4KDa-FITC dextran translocation observed for *R. hominis* and *E. eligens* was similar to the positive control of a stable analog of glucagon-like peptide 2 (Gly2-GLP2; p = 0.48), which is known to reduce the severity of colonic injury (see, e.g., Drucker D.J., et al. (1999) Am J Physiol 276;G79-91) and has been recently shown to improve glycemic control (see, e.g., Amato et al., (2016) J Endocrinol 229:R57-R66). Results are presented as mean ± SEM and statistical analysis was performed by a one-way ANOVA (n = 10).

### B. Effects of R. hominis and E. eligens on serum LBP levels

Animals treated as described in Example 4 were also assessed for levels of Lipopolysaccharide binding protein (LBP) was measured in serum. A reduction in LBP in the serum of DSS-treated animals can be a sign of epithelial barrier repair. Following 6 days of treatment, mice were euthanized, blood was collected, and LBP was measured in serum by ELISA. LBP plays a key role in activating the innate immune response to bacterial challenge (see, e.g., Ding P.H. and Jin L.J. (2014) J Periodontal Res. 49:1-9; Schröder N.W., et al. (2004) J immunol 173:2683-91.)

Results are shown in **FIG. 6** and are presented as mean ± SEM. A significant increase in LBP concentration was observed in response to DSS (p < 0.0001). A reduction in LBP was observed in *R. hominis* and *E. eligens* treated mice given DSS as compared to DSS mice treated with vehicle (p = 0.07). Results are presented as mean ± SEM and statistical analysis was performed by a one-way ANOVA (n = 10). Inflammatory responses as reported by LBP are therefore reduced by the administration of a composition comprising *R. hominis* and *E. eligens.*

### C. Effects of R. hominis and E. eligens on body weight

An epithelial barrier function disorder and associated inflammatory bowel disease can result in a detrimental loss in weight. Mice treated as described in Example 4 were also assessed for effects of treatment with *R. hominis* and *E. eligens* on body weight.

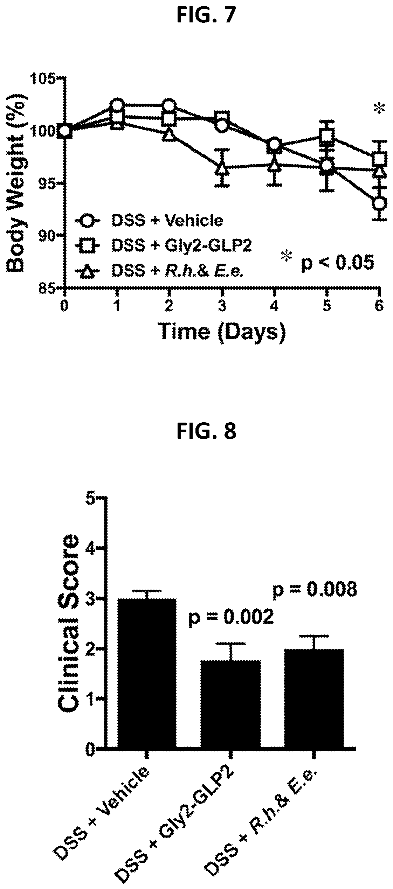

Body weight was measured daily for mice included in this DSS model study. Results are shown in **FIG. 7** and are presented as mean ± SEM. Percent change from starting weight on Day Zero ("0") was determined for each mouse. *R. hominis* and *E. eligens* administration to DSS treated mice significantly improved body weight as compared to vehicle treated DSS mice (p = 0.05). Weight loss in mice treated with *R. hominis* and *E. eligens* at Day 6 was similar to weight loss observed with Gly2-GLP2 (p = 0.51). Statistical differences between groups were determined by a repeated measures 2-way ANOVA (n = 10).

### D. Effects of R. hominis and E. eligens on gross pathology

Mice treated as described in Example 4 were also assessed for effects of treatment with *R. hominis* and *E. eligens* on gross pathology of the intestine. Results are shown in **FIG. 8** and are presented as mean ± SEM.

*R. hominis* and *E. eligens* administration to DSS treated mice significantly improved gross pathology as compared to vehicle treated DSS mice (p = 0.008). However, no differences in clinical scores were observed between mice given DSS and treated with either Gly2-GLP2 or *R. hominis* and *E*. *eligens* (p = 0.54). The clinical scoring system used was: (0) = no gross pathology, (1) = streaks of blood visible in feces, (2) = completely bloody fecal pellets, (3) = bloody fecal material visible in cecum, (4) = bloody fecal material in cecum and loose stool, (5) = rectal bleeding. Statistical differences between groups were determined by a one-way ANOVA (n = 10).

### E. Effects of R. hominis and E. eligens on colon weight to length ratio

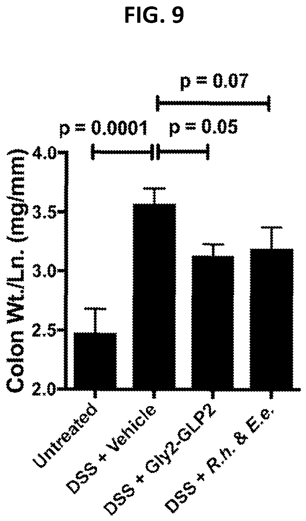

DSS treatment induces a shortening of the colonic tissue while at the same time inducing edema with resultant increased colon weight. Accordingly, DSS treatment results in an increase in the weight to length (Wt./Ln.) ratio of the colonic tissue. The mice treated as described in Example 4 where therefore assessed with respect to colon Wt./Ln. ratio. Colon length and weight were measured in in these mice and results are shown in **FIG. 9** and are presented as mean ± SEM.

*R. hominis* and *E. eligens* administration to DSS treated mice prevented the increase in colon Wt./Ln. ratio elicited by DSS, although the difference was not statistically significant (p = 0.07). Statistical analysis was performed using a one-way ANOVA (n = 10).

### Example 5

### Effects of R. hominis and E. eligens treatment in ob/ob mice on epithelial barrier function readouts, inflammation centric barrier function readouts, and maintenance of small intestine (SI) and colon length.

Experiments were performed to study the effects of *R. hominis* and *E. eligens* administration on intestinal health and function in *ob*/*ob* mice. The *ob*/*ob* mice were treated as described in Example 1 above.

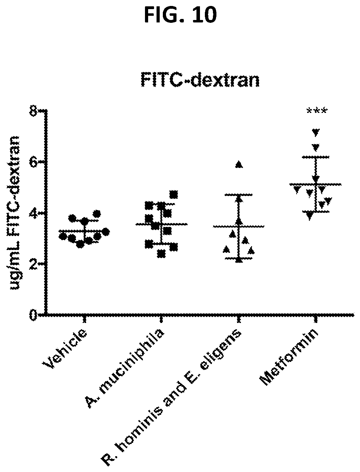

Following 2 weeks of treatment with *R. hominis* and *E. eligens,* mice were tested for intestinal permeability (see method of Example 4 above). After a 4-5 hour fast, mice were gavaged with 200mg/kg FITC-dextran (4kD). One hour after the gavage, peripheral blood was collected. Gut permeability was measured by fluorescence intensity in the serum. Results are illustrated in **FIG. 10** and show that treatment with *A. muciniphila* or with *R. hominis* and *E*. *eligens* had no effect on gut permeability while metformin treated resulted in a significant increase in gut permeability compared to animals treated with vehicle.

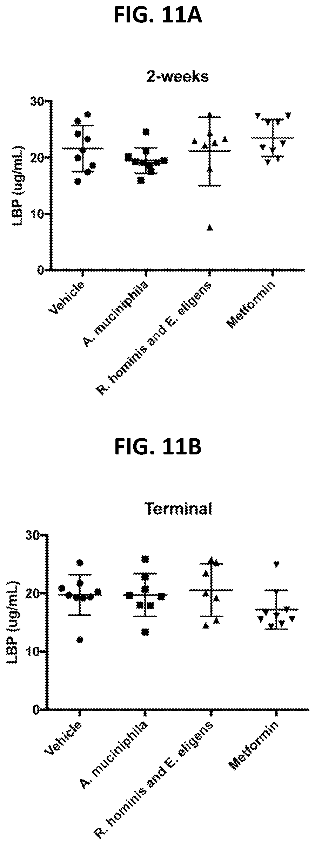

Lipopolysaccharide (LPS) binding protein (LBP) was measured after 2 weeks of treatment and at the termination of the study (3 weeks). LBP, a marker of inflammation and LPS exposure, was measured in the serum by ELISA. As shown in **FIG. 11A** (Day 15) and **FIG. 11B** (Day 22), none of the treatments affected LBP

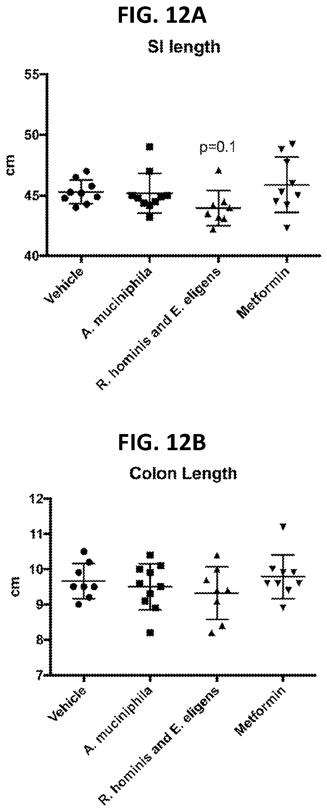

At the termination of the study (Day 22), the length of the small intestine and the length of the colon was measured for each mouse to determine if bacterial treatment affected intestinal health. Results are shown in **FIG. 12A** (small intestine) and **FIG. 12B** (colon). None of the treatments affected colon or small intestine length as compared to animals treated with vehicle only.

### Example 6

### R. hominis and E. eligens for treatment of type 2 diabetes

A clinical trial to study the effects of a live biotherapeutic product which contains both *Roseburia hominis* and *Eubacterium eligens* is carried out to studied the effects of this composition for treating type 2 diabetes. The study includes measurements of the *R. hominis* and *E. eligens* bacteria in fecal matter, glucose tolerance and insulin sensitivity before and after treatment.

The study is a randomized, placebo-controlled, double-blinded trial. Twenty-four adults diagnosed with Type 2 diabetes are randomly assigned to a test group (n=12) and a control group (n=12). Each subject in the test group is orally administered a capsule containing equal amounts of *R. hominis* and *E. eligens* (~10⁵ CFU each) while the subjects in the control group receive a placebo capsule. Each subject in the test group is orally administered a capsule containing. Each subject is the test group is orally administered a placebo tablet. Capsules are administered to each subject q.d. in the morning for a period of 4 weeks. The subjects are instructed to abstain from fermented dairy products during the weeks of treatment. Strenuous physical exercise is avoided for 48 hours before the oral glucose tolerance test (OGTT). Oral anti-diabetics and statins are withheld for 1 week and all other medications for 24 hours.

Stool samples are collected within 24 hours prior to the first administration of a capsule and on the last day of administration. Samples are kept at 5°C for no longer than 24 hours, and are stored at -80°C until analyzed in a laboratory. Total bacterial DNA and genomic DNA is extracted from the fecal samples.

To determine the levels of *R. hominis* and *E. eligens* in the stool samples, gene sequences specific for *R. hominis* and *E. eligens* are PCR-amplified, confirmed by DNA sequencing, then analyzed by real-time PCR to determine the levels of *R. hominis* and *E. eligens* in the stool samples of each subject.

OGTTs are administered at the start and end of the study to determine effects of the treatment of glucose tolerance. Each subject is given an oral glucose tolerance test (OGTT) the day prior to the first oral administration of the capsule and on the last day of administration. Subjects drink 75 g glucose diluted in 500 ml water over 5 minutes. Plasma glucose is measured at baseline, and at 1 and 2 hours after the glucose administration.

The effects of treatment on insulin resistance is measured using the hyperinculinemic-euglycemic clamp technique. Generally, after an overnight fast, subjects report to the laboratory and an intravenous catheter for administration of insulin, glucose and electrolyes is place in an antecubital vein. A retrograde catheter is inserted into a dorsal vein in the contralateral hand.

The duration of the clamp is 180 minutes. Insulin (100 IU/ml) is infused continuously at a rate of 0.120 IU/min per m². Plasma glucose and K are measured at 5 minute intervals during hour 1 and at 10 minute intervals during the hours 2 and 3. Plasma glucose levels are targeted at 5.0±0.2 mmol/l by adjusting the infusion rates of glucose (200 g/l) on a computer-controlled infusion pump. M, the whole-body metabolic rate of glucose, is calculated from the glucose infusion rate in the interval between 120 and 180 min according to the equations of DeFronzo et al. (1979, Am J Physiol 237:E214-E223). Isotonic saline with K is continuously infused to avoid hypokalemia. Blood for analysis of insulin is drawn every 30 minutes into EDTA-containing tubes and centrifuged instantly. Plasma is stored at -80°C until analysis.

### Example 7

### R. hominis and E. eligens for treatment of ulcerative colitis

A randomized, placebo-controlled trial is performed in order to determine the therapeutic effects of a composition containing *R. hominis* and *E. eligens* when orally administered to subjects diagnosed with moderately to severely active ulcerative colitis (as determined by endoscopic evidence; Mayo score of ≥ 6 and a Mayo endoscopic subscore ≥ 2 within the 2 weeks prior to study composition administration. 250 adult subjects are selected who had an inadequate response to one or more of oral aminosalicylates, prednisone, immunosuppressants, intravenous (IV) hydrocortisone, or an anti-TNF agent; were intolerant to one or more of the above; and/or were currently receiving oral aminosalicylates, prednisone or azathioprine, or 6-mercaptopurine.

Twenty-four adults diagnosed with Type 2 diabetes are randomly assigned to a test group (n=12) and a control group (n=12). Each subject in the test group is orally administered a capsule containing equal amounts of *R. hominis* and *E. eligens* (~10⁵ to 10⁸ CFU each) while the subjects in the control group receive a placebo capsule. Each subject in the test group is orally administered a capsule containing. Each subject is the test group is orally administered a placebo tablet. Capsules are administered to each subject q.d. in the morning for a period of 12 weeks.

The primary endpoint is the proportion of subjects in the treatment group in clinical remission, defined as a Mayo score < 2 points with no individual subscore > 1 point, at Week 12. Key secondary endpoints at Week 12 are clinical response (reduction from baseline ≥ 3 points and ≥ 30% in Mayo score, reduction ≥ 1 in rectal bleeding subscore, or absolute rectal bleeding subscore ≤ 1), mucosal healing (endoscopy subscore ≤ 1), and Inflammatory Bowel Disease Questionnaire (IBDQ) response (≥ 16-point change from baseline).

The Mayo score is a composite index of four disease variables (stool frequency, rectal bleeding, endoscopy findings, and physician global assessment), each scored on a scale of 0 to 3, with higher scores indicating greater frequency or severity (total score: 0 to 12) (Schroeder et al., 1987, N Engl J Med, 317:1625-1629; Rutgeerts et al., 2005, N Engl J Med, 353:2462-2476). Partial Mayo scores (endoscopy subscore omitted) range from 0 to 9. Mayo scores (including endoscopy) are assessed at baseline and Week 12; partial Mayo scores are recorded at Weeks 1, 2, 5, 7, and 12. Patient diaries are used to assist in Mayo score calculations.

The IBDQ is a self-administered 32-item questionnaire that evaluates quality of life across four dimensions (bowel, systemic symptoms, emotional function, and social function), with responses ranging from 1 (severe impact) to 7 (normal health). Total IBDQ scores range from 32 to 224, with higher scores indicating better quality of life (Guyatt et al., 1989, Gastroenterology, 96:804-810; Mitchell et al., 1988, J Clin Gastroenterol, 10:306-310). The IBDQ is administered at baseline and Week 12. IBDQ response is defined as a ≥ 16 point increase in IBDQ.

Blood for assessment of hsCRP is drawn at baseline and Weeks 1, 3, 5, 7, 9, and 12. Fecal calprotectin is assessed in stool samples at baseline and Week 12. Histological assessment of gut mucosa following biopsy is performed using the Geboes Index, a seven-component index where 0 (lack of architectural change or other histological abnormalities) indicates the least severe damage and 4 (crypt destruction) indicates the most severe damage.

## Claims

1. A composition comprising:
i. a therapeutically effective amount of an isolated *Roseburia hominis* (*R. hominis*) bacterial strain;
ii. a therapeutically effective amount of an isolated *Eubacterium eligens* (*E. eligens*) bacterial strain; and
iii. a pharmaceutically acceptable carrier,
for use in a method for treating type 2 diabetes in a subject in need thereof, said method comprising administering to the subject said composition.

2. The composition for use of claim 1, wherein the *R. hominis* and *E*. *eligens* are each viable.

3. The composition for use of claim 1, wherein the subject has a fasting blood glucose level of greater than about 125 mg/dL or greater than about 130 mg/dL.

4. The composition for use of claim 1, wherein the subject has a 2-hour value for a 75 gram oral glucose tolerance test of greater than about 140 mg/dL.

5. The composition for use of claim 1, wherein the therapeutically effective amount of *R. hominis* comprises about 1x10⁷ to 1x10¹² colony forming units (CFU) of *R. hominis.*

6. The composition for use of claim 1, wherein the therapeutically effective amount of *E. eligens* comprises about 1x10⁷ to 1x10¹² colony forming units (CFU) of *E. eligens.*

7. The composition for use of claim 1, wherein the method comprises administering the composition to the subject once, twice or three times per day over a time period of at least about 1-52 weeks.

8. The composition for use of claim 1, wherein the method results in a reduction of the subject's fasting blood glucose level of at least about 5%, 10%, 20%, 30% or 40% of the fasting blood glucose level of the subject prior to the first administration of the composition.

9. The composition for use of claim 1 wherein the reduction of the subject's fasting blood glucose level is measured at 1 month, 2 months, 3 months, 4 months, 5 months, 6 months, 7 months, 8 months, 9 months, 10 months, 11 months, or 12 months after the first administration of the composition.

10. The composition for use of claim 1, wherein the *Roseburia hominis* bacterial strain has a 16S rRNA sequence that is at least 97% identical to SEQ ID NO: 1, a 16S rRNA sequence that is at least 97% identical to SEQ ID NO:2, a 16S rRNA sequence that is at least 97% identical to SEQ ID NO:3, and/or a 16S rRNA sequence that is at least 97% identical to SEQ ID NO:4.

11. The composition for use of claim 1, wherein the *Eubacterium eligens* bacterial strain has a 16S rRNA sequence that is at least 97% identical to SEQ ID NO: 5, a 16S rRNA sequence that is at least 97% identical to SEQ ID NO: 6, a 16S rRNA sequence that is at least 97% identical to SEQ ID NO:7, a 16S rRNA sequence that is at least 97% identical to SEQ ID NO:8, a 16S rRNA sequence that is at least 97% identical to SEQ ID NO:9, and/or a 16S rRNA sequence that is at least 97% identical to SEQ ID NO:10.

12. The composition for use of claim 1, wherein the *Roseburia hominis* bacterial strain has a 16S rRNA sequence that is at least 98% identical to SEQ ID NO: 1, a 16S rRNA sequence that is at least 98% identical to SEQ ID NO:2, a 16S rRNA sequence that is at least 98% identical to SEQ ID NO:3, and/or a 16S rRNA sequence that is at least 98% identical to SEQ ID NO:4,
optionally wherein the *Roseburia hominis* bacterial strain has a 16S rRNA sequence that is at least 99% identical to SEQ ID NO: 1, a 16S rRNA sequence that is at least 99% identical to SEQ ID NO:2, a 16S rRNA sequence that is at least 99% identical to SEQ ID NO:3, and/or a 16S rRNA sequence that is at least 99% identical to SEQ ID NO:4.

13. The composition for use of claim 1, wherein the *Roseburia hominis* bacterial strain has a 16S rRNA sequence that is 100% identical to SEQ ID NO: 1, a 16S rRNA sequence that is 100% identical to SEQ ID NO:2, a 16S rRNA sequence that is 100% identical to SEQ ID NO:3, and/or a 16S rRNA sequence that is 100% identical to SEQ ID NO:4.

14. The composition for use of claim 1, wherein the *Eubacterium eligens* bacterial strain has a 16S rRNA sequence that is at least 98% identical to SEQ ID NO: 5, a 16S rRNA sequence that is at least 98% identical to SEQ ID NO: 6, a 16S rRNA sequence that is at least 98% identical to SEQ ID NO:7, a 16S rRNA sequence that is at least 98% identical to SEQ ID NO:8, a 16S rRNA sequence that is at least 98% identical to SEQ ID NO:9, and/or a 16S rRNA sequence that is at least 98% identical to SEQ ID NO: 10, optionally wherein the *Eubacterium eligens* bacterial strain has a 16S rRNA sequence that is at least 99% identical to SEQ ID NO: 5, a 16S rRNA sequence that is at least 99% identical to SEQ ID NO: 6, a 16S rRNA sequence that is at least 99% identical to SEQ ID NO:7, a 16S rRNA sequence that is at least 99% identical to SEQ ID NO:8, a 16S rRNA sequence that is at least 99% identical to SEQ ID NO:9, and/or a 16S rRNA sequence that is at least 99% identical to SEQ ID NO: 10.

15. The composition for use of claim 1, wherein the *Eubacterium eligens* bacterial strain has a 16S rRNA sequence that is 100% identical to SEQ ID NO: 5, a 16S rRNA sequence that is 100% identical to SEQ ID NO: 6, a 16S rRNA sequence that is 100% identical to SEQ ID NO:7, a 16S rRNA sequence that is 100% identical to SEQ ID NO:8, a 16S rRNA sequence that is 100% identical to SEQ ID NO:9, and/or a 16S rRNA sequence that is 100% identical to SEQ ID NO: 10.

## Patentansprüche

1. Zusammensetzung, umfassend:
i. eine therapeutisch wirksame Menge eines isolierten Bakterienstamms von Roseburia hominis (R. hominis);
ii. eine therapeutisch wirksame Menge eines isolierten Bakterienstamms von Eubacterium eligens (E. eligens);
iii. einen pharmazeutisch verträglichen Träger,
zur Verwendung in einem Verfahren zur Behandlung von Typ-2-Diabetes bei einem Patienten, der eine solche Behandlung benötigt, wobei das Verfahren das Verabreichen der Zusammensetzung an den Patienten umfasst.

2. Zusammensetzung zur Verwendung nach Anspruch 1, wobei R. hominis und E. eligens jeweils lebensfähig sind.

3. Zusammensetzung zur Verwendung nach Anspruch 1, wobei der Patient einen Nüchternblutzuckerspiegel von mehr als etwa 125 mg/dl oder mehr als etwa 130 mg/dl hat.

4. Zusammensetzung zur Verwendung nach Anspruch 1, wobei der Patient einen 2-Stunden-Wert für einen 75-Gramm-Glukosetoleranztest von mehr als etwa 140 mg/dl hat.

5. Zusammensetzung zur Verwendung nach Anspruch 1, wobei die therapeutisch wirksame Menge an R. hominis etwa 1x107 bis 1x1012 koloniebildende Einheiten (CFU) von R. hominis umfasst.

6. Zusammensetzung zur Verwendung nach Anspruch 1, wobei die therapeutisch wirksame Menge an E. eligens etwa 1x107 bis 1x1012 koloniebildende Einheiten (CFU) von E. eligens umfasst.

7. Zusammensetzung zur Verwendung nach Anspruch 1, wobei das Verfahren das Verabreichen der Zusammensetzung an den Patienten einmal, zweimal oder dreimal pro Tag über einen Zeitraum von mindestens etwa 1 bis 52 Wochen umfasst.

8. Zusammensetzung zur Verwendung nach Anspruch 1, wobei das Verfahren zu einer Verringerung des Nüchternblutzuckerspiegels des Patienten um mindestens etwa 5 %, 10 %, 20 %, 30 % oder 40 % des Nüchternblutzuckerspiegels des Patienten vor der ersten Verabreichung der Zusammensetzung führt.

9. Zusammensetzung zur Verwendung nach Anspruch 1, wobei die Verringerung des Nüchternblutzuckerspiegels des Patienten 1 Monat, 2 Monate, 3 Monate, 4 Monate, 5 Monate, 6 Monate, 7 Monate, 8 Monate, 9 Monate, 10 Monate, 11 Monate oder 12 Monate nach der ersten Verabreichung der Zusammensetzung gemessen wird.

10. Zusammensetzung zur Verwendung nach Anspruch 1, wobei der Bakterienstamm Roseburia hominis eine 16S-rRNA-Sequenz hat, die zu mindestens 97 % identisch ist mit SEQ ID NR: 1, eine 16S-rRNA-Sequenz, die zu mindestens 97 % identisch ist mit SEQ ID NR:2, eine 16S-rRNA-Sequenz, die zu mindestens 97 % identisch ist mit SEQ ID NR:3, und/oder eine 16S-rRNA-Sequenz, die zu mindestens 97 % identisch ist mit SEQ ID NR:4.

11. Zusammensetzung zur Verwendung nach Anspruch 1, wobei der Bakterienstamm Eubacterium eligens eine 16S-rRNA-Sequenz hat, die zu mindestens 97 % identisch ist mit SEQ ID NR: 5, eine 16S-rRNA-Sequenz, die zu mindestens 97 % identisch ist mit SEQ ID NR: 6, eine 16S-rRNA-Sequenz, die zu mindestens 97 % identisch ist mit SEQ ID NR:7, eine 16S-rRNA-Sequenz, die zu mindestens 97 % identisch ist mit SEQ ID NR: 8, eine 16S-rRNA-Sequenz, die zu mindestens 97 % identisch ist mit SEQ ID NR:9, und/oder eine 16S-rRNA-Sequenz, die zu mindestens 97 % identisch ist mit SEQ ID NR: 10.

12. Zusammensetzung zur Verwendung nach Anspruch 1, wobei der Bakterienstamm Roseburia hominis eine 16S rRNA-Sequenz hat, die zu mindestens 98 % identisch ist mit SEQ ID NR: 1, eine 16S-rRNA-Sequenz, die zu mindestens 98 % identisch ist mit SEQ ID NR:2, eine 16S-rRNA-Sequenz, die zu mindestens 98 % identisch ist mit SEQ ID NR:3, und/oder eine 16S-rRNA-Sequenz, die zu mindestens 98 % identisch ist mit SEQ ID NR:4,
optional wobei der Bakterienstamm Roseburia hominis eine 16S rRNA-Sequenz hat, die zu mindestens 99 % identisch ist mit SEQ ID NR: 1, eine 16S-rRNA-Sequenz, die zu mindestens 99 % identisch ist mit SEQ ID NR:2, eine 16S-rRNA-Sequenz, die zu mindestens 99 % identisch ist mit SEQ ID NR:3, und/oder eine 16S-rRNA-Sequenz, die zu mindestens 99 % identisch ist mit SEQ ID NR:4.

13. Zusammensetzung zur Verwendung nach Anspruch 1, wobei der Bakterienstamm Roseburia hominis eine 16S rRNA-Sequenz hat, die zu 100 % identisch ist mit SEQ ID NR: 1, eine 16S-rRNA-Sequenz, die zu 100 % identisch ist mit SEQ ID NR:2, eine 16S-rRNA-Sequenz, die zu 100 % identisch ist mit SEQ ID NR:3, und/oder eine 16S-rRNA-Sequenz, die zu 100 % identisch ist mit SEQ ID NR:4.

14. Zusammensetzung zur Verwendung nach Anspruch 1, wobei der Bakterienstamm Eubacterium eligens eine 16S-rRNA-Sequenz hat, die zu mindestens 98 % identisch ist mit SEQ ID NR: 5, eine 16S-rRNA-Sequenz, die zu mindestens 98 % identisch ist mit SEQ ID NR: 6, eine 16S-rRNA-Sequenz, die zu mindestens 98 % identisch ist mit SEQ ID NR:7, eine 16S-rRNA-Sequenz, die zu mindestens 98 % identisch ist mit SEQ ID NR: 8, eine 16S-rRNA-Sequenz, die zu mindestens 98 % identisch ist mit SEQ ID NR:9, und/oder eine 16S-rRNA-Sequenz, die zu mindestens 98 % identisch ist mit SEQ ID NR: 10, wobei optional der Bakterienstamm Eubacterium eligens eine 16S-rRNA-Sequenz hat, die zu mindestens 99 % identisch ist mit SEQ ID NR: 5, eine 16S-rRNA-Sequenz, die zu mindestens 99 % identisch ist mit SEQ ID NR: 6, eine 16S-rRNA-Sequenz, die zu mindestens 99 % identisch ist mit SEQ ID NR:7, eine 16S-rRNA-Sequenz, die zu mindestens 99 % identisch ist mit SEQ ID NR: 8, eine 16S-rRNA-Sequenz, die zu mindestens 99 % identisch ist mit SEQ ID NR:9, und/oder eine 16S-rRNA-Sequenz, die zu mindestens 99 % identisch ist mit SEQ ID NR: 10.

15. Zusammensetzung zur Verwendung nach Anspruch 1, wobei der Bakterienstamm Eubacterium eligens eine 16S-rRNA-Sequenz hat, die zu 100 % identisch ist mit SEQ ID NR: 5, eine 16S-rRNA-Sequenz, die zu 100 % identisch ist mit SEQ ID NR: 6, eine 16S-rRNA-Sequenz, die zu 100 % identisch ist mit SEQ ID NR:7, eine 16S-rRNA-Sequenz, die zu 100 % identisch ist mit SEQ ID NR: 8, eine 16S-rRNA-Sequenz, die zu 100 % identisch ist mit SEQ ID NR:9, und/oder eine 16S-rRNA-Sequenz, die zu 100 % identisch ist mit SEQ ID NR: 10.

## Revendications

1. Composition, comprenant :
i. une quantité thérapeutiquement efficace d'une souche bactérienne isolée de Roseburia hominis (R. hominis) ;
ii. une quantité thérapeutiquement efficace d'une souche bactérienne isolée d'Eubacterium eligens (E. eligens) ; et
iii. un support pharmaceutiquement acceptable,
pour utilisation dans un procédé de traitement du diabète de type 2 chez un sujet qui en a besoin, ledit procédé comprenant l'administration au sujet de ladite composition.

2. Composition pour utilisation selon la revendication 1, dans laquelle R. hominis et E. eligens sont tous deux viables.

3. Composition pour utilisation selon la revendication 1, dans laquelle le sujet a une glycémie à jeun supérieure à environ 125 mg/dL ou supérieure à environ 130 mg/dL.

4. Composition pour utilisation selon la revendication 1, dans laquelle le sujet a une valeur à 2 heures pour un test de tolérance au glucose oral de 75 grammes supérieure à environ 140 mg/dL.

5. Composition pour utilisation selon la revendication 1, dans laquelle la quantité thérapeutiquement efficace de R. hominis comprend environ 1x107 à 1x1012 unités formant colonies (UFC) de R. hominis.

6. Composition pour utilisation selon la revendication 1, dans laquelle la quantité thérapeutiquement efficace d'E. eligens comprend 1x107 à 1x1012 unités formant colonies (UFC) d'E. eligens.

7. Composition pour utilisation selon la revendication 1, dans laquelle le procédé comprend l'administration de la composition au sujet une, deux ou trois fois par jour sur une période de temps d'au moins environ 1 à 52 semaines.

8. Composition pour utilisation selon la revendication 1, dans laquelle le procédé résulte en une réduction de la glycémie à jeun du sujet d'au moins 5 %, 10 %, 20 %, 30 % ou 40 % par rapport à la glycémie à jeun du sujet avant la première administration de la composition.

9. Composition pour utilisation selon la revendication 1, dans laquelle la réduction de la glycémie à jeun du sujet est mesurée 1 mois, 2 mois, 3 mois, 4 mois, 5 mois, 6 mois, 7 mois, 8 mois, 9 mois, 10 mois, 11 mois ou 12 mois après la première administration de la composition.

10. Composition pour utilisation selon la revendication 1, dans laquelle la souche bactérienne de Roseburia hominis a une séquence d'ARNr 16S qui est identique à au moins 97 % à SEQ ID NO : 1, une séquence d'ARNr 16S qui est identique à au moins 97 % à SEQ ID NO : 2, une séquence d'ARNr 16S qui est identique à au moins 97 % à SEQ ID NO : 3, et/ou une séquence d'ARNr 16S qui est identique à au moins 97 % à SEQ ID NO : 4.

11. Composition pour utilisation selon la revendication 1, dans laquelle la souche bactérienne d'Eubacterium eligens a une séquence d'ARNr 16S qui est identique à au moins 97 % à SEQ ID NO : 5, une séquence d'ARNr 16S qui est identique à au moins 97 % à SEQ ID NO : 6, une séquence d'ARNr 16S qui est identique à au moins 97 % à SEQ ID NO : 7, une séquence d'ARNr 16S qui est identique à au moins 97 % à SEQ ID NO : 8, une séquence d'ARNr 16S qui est identique à au moins 97 % à SEQ ID NO : 9, et/ou une séquence d'ARNr 16S qui est identique à au moins 97 % à SEQ ID NO : 10.

12. Composition pour utilisation selon la revendication 1, dans laquelle la souche bactérienne de Roseburia hominis à une séquence d'ARNr 16S qui est identique à au moins 98 % à SEQ ID NO : 1, une séquence d'ARNr 16S qui est identique à au moins 98 % à SEQ ID NO : 2, une séquence d'ARNr 16S qui est identique à au moins 98 % SEQ ID NO : 3, et/ou une séquence d'ARNr 16S qui est identique à au moins 98 % à SEQ ID NO : 4,
éventuellement dans laquelle la souche bactérienne de Roseburia hominis a une séquence d'ARNr 16S qui est identique à au moins 99 % à SEQ ID NO : 1, une séquence d'ARNr 16S qui est identique à au moins 99 % à SEQ ID NO : 2, une séquence d'ARNr 16S qui est identique à au moins 99 % à SEQ ID NO : 3, et/ou une séquence d'ARNr 16S qui est identique à au moins 99 % à SEQ ID NO : 4.

13. Composition pour utilisation selon la revendication 1, dans laquelle la souche bactérienne de Roseburia hominis a une séquence d'ARNr 16S qui est identique à 100 % à SEQ ID NO : 1, une séquence d'ARNr 16S qui est identique à 100 % à SEQ ID NO : 2, une séquence d'ARNr 16S qui est identique à 100 % à SEQ ID NO : 3, et/ou une séquence d'ARNr 16S qui est identique à 100 % à SEQ ID NO : 4.

14. Composition pour utilisation selon la revendication 1, dans laquelle la souche bactérienne d'Eubacterium eligens a une séquence d'ARNr 16S qui est identique à au moins 98 % à SEQ ID NO : 5, une séquence d'ARNr 16S qui est identique à au moins 98 % à SEQ ID NO : 6, une séquence d'ARNr 16S qui est identique à au moins 98 % à SEQ ID NO : 7, une séquence d'ARNr 16S qui est identique à au moins 98 % à SEQ ID NO : 8, une séquence d'ARNr 16S qui est identique à au moins 98 % à SEQ ID NO : 9, et/ou une séquence d'ARNr 16S qui est identique à au moins 98 % à SEQ ID NO : 10, éventuellement dans laquelle la souche bactérienne d'Eubacterium eligens a une séquence d'ARNr 16S qui est identique à au moins 99 % à SEQ ID NO : 5, une séquence d'ARNr 16S qui est identique à au moins 99 % à SEQ ID NO : 6, une séquence d'ARNr 16S qui est identique à au moins 99 % à SEQ ID NO : 7, une séquence d'ARNr 16S qui est identique à au moins 99 % à SEQ ID NO : 8, une séquence d'ARNr 16S qui est identique à au moins 99 % à SEQ ID NO : 9, et/ou une séquence d'ARNr 16S qui est identique à au moins 99 % à SEQ ID NO : 10.

15. Composition pour utilisation selon la revendication 1, dans laquelle la souche bactérienne d'Eubacterium eligens a une séquence d'ARNr 16S qui est identique à 100 % à SEQ ID NO : 5, une séquence d'ARNr 16S qui est identique à 100 % à SEQ ID NO : 6, une séquence d'ARNr 16S qui est identique à 100 % à SEQ ID NO : 7, une séquence d'ARNr 16S qui est identique à 100 % à SEQ ID NO : 8, une séquence d'ARNr 16S qui est identique à 100 % à SEQ ID NO : 9, et/ou une séquence d'ARNr 16S qui est identique à 100 % à SEQ ID NO : 10.
